(19)

**Europäisches Patentamt**

**European Patent Office**

**Office européen des brevets**

(11) **EP 2 108 954 A1**

(12) **EUROPEAN PATENT APPLICATION**

(43) Date of publication:
**14.10.2009 Bulletin 2009/42**

(51) Int Cl.:
***G01N 33/50*** (2006.01)   ***G01N 33/566*** (2006.01)

(21) Application number: **08007140.0**

(22) Date of filing: **10.04.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Max-Planck-Gesellschaft zur Förderung der Wissenschaften e.V.
80539 München (DE)**

(72) Inventors:
• **Wicher, Dieter
07749 Jena (DE)**
• **Hansson, Bill
07749 Jena (DE)**

(74) Representative: **Wichmann, Hendrik
Isenbruck Bösl Hörschler Wichmann Huhn LLP
Prinzregentenstrasse 68
81675 München (DE)**

(54) **A test system for detecting intracellular cyclic nucleotide level and methods and uses thereof**

(57)     The present invention relates to a test system for detecting a cyclic nucleotide, the test system comprising (i) a cell comprising an insect cyclic nucleotide-activated ion channel protein or a derivative thereof and (ii) means for detecting the activity of the ion channel protein, methods for detecting a change of an intracellular cyclic nucleotide level, methods of identifying an agent capable of modifying an intracellular cyclic nucleotide level, a kit comprising the cell or means for producing the cell and means for detecting activity and the use of the test system for detecting a change of an intracellular cyclic nucleotide level or for identifying an agent capable of modifying intracellular cyclic nucleotide level.

**EP 2 108 954 A1**

**Description**

The present invention relates to a test system for detecting a cyclic nucleotide, the test system comprising (i) a cell comprising an insect cyclic nucleotide-activated ion channel protein or a derivative thereof and (ii) means for detecting the activity of the ion channel protein, methods for detecting a change of an intracellular cyclic nucleotide level, methods of identifying an agent capable of modifying an intracellular cyclic nucleotide level, a kit comprising the cell or means for producing the cell and means for detecting activity and the use of the test system for detecting a change of an intracellular cyclic nucleotide level or for identifying an agent capable of modifying intracellular cyclic nucleotide level.

In order to perceive and respond to their environment, cells have to process information from outside the cell to its inside. The information may be a chemical signal (first messenger) such as a hormone, neurotransmitter, pheromone, odorant etc., a physical signal (temperature) or a state (starvation, shear stress). Particularly, chemical signals may not be able to penetrate through the cell membrane. Accordingly, the information has to be transduced from outside to inside which may be accomplished by a class of proteins, named receptors. They are capable of exerting a reaction upon binding of the corresponding ligand, i.e. the first messenger. This receptor-mediated signal may then induce a further intracellular chemical signal, i.e. the second messenger.

Due to their number and involvement in physiological and pathophysiological processes, the most important class of receptors are G protein-coupled receptors (GPCRs), also known as seven transmembrane domain receptors, 7TM receptors, heptahelical receptors, and G protein-linked receptors (GPLR). This receptor family encompasses integral membrane proteins having seven hydrophobic alpha helical transmembrane domains with three intracellular and three extracellular loops, an extracellular N-terminus and an intracellular C-terminus. A great number of different GPCRs are found in all tissues and exert a great variety of functions, such as receptors for odorants, light, and taste, as well as receptors for inflammatory molecules (e.g. chemokines, histamines), hormones (e.g. adrenaline, glucagon, somatostatin, follicle-stimulating hormone, atriopeptin) and neurotransmitters (e.g. noradrenaline, dopamine, gamma-aminobutyric acid (GABA), acetylcholine).

Upon binding of an agonist, the GPCR couples to a heterotrimeric G-protein. The G-protein complex consists of three polypeptide subunits, alpha, beta, and gamma. Subsequent to binding of a ligand to the GPCR, the affinity for guanosine diphosphate (GDP) bound to the alpha subunit of the G-protein complex is decreased and is exchanged against a guanosine triphosphate (GTP). The thereby activated alpha subunit dissociates from the dimeric beta-gamma subunit and transduces the signal by activating an effector, e.g. by activating an adenylyl cyclase which then generates the second messenger cAMP. Amongst others, this second messenger may then activate one or more further cell proteins which trigger the cell's response to the external signal.

The detection of changes in the intracellular cyclic nucleotide concentration has a high relevance in scientific and pharmaceutical research. In addition to their normal function, there are several disorders known to be associated with or which can be treated by targeting signal transduction cascades, particularly those involving GPCRs, e.g. cardiovascular diseases, neurological disorders, allergies, asthma, Parkinson's disease, osteoporosis or hypotension. It is estimated that nearly one third of all prescription drugs are GPCR ligands.

Thus, there is a need for sensitive assays which can detect changes in the signal transduction cascades, particularly intracellular cyclic nucleotides (which constitute one important class of second messengers) upon interaction of a cell with a known ligand.

A further target is the identification of new ligands for GPCRs. It is of particular interest to identify for example highly selective ligands, highly effective ligands and/or ligands with high affinity for a GPCR. Additionally, there is an interest of identifying ligands for so-called orphan receptors, whose ligands are not yet known. One way of identifying such ligands, agonists and antagonists may be by screening substance libraries in high-throughput assays.

There exist a number of assays for the detection of changes of the intracellular cyclic nucleotide concentration which are either sensitive, but not suitable for high-throughput assays, or suitable for high-throughput assays, but rather insensitive.

For example, the transcription reporter assay (using cAMP response element (CRE) containing promoters linked with a reporter gene, such as luciferase), a cAMP-immunoassay (Applied Biosystems), an enzyme assay for adenylyl cyclase (Molecular Devices), a cAMP- fluorescence assay (Perkin Elmer), or a cAMP-radioreceptor assay may be used. However, all these assays use cell lysates and are therefore end point assays.

For use in an assay for the detection of changes of the intracellular cyclic nucleotide concentration in a living cell, cyclic nucleotide-gated (CNG) channels may be employed. This channel is activated by an increase of the intracellular cyclic nucleotide concentration. Methods which comprise the use of CNG channels are disclosed in WO 2005/043158, US 2005/0221426, and US 2003/0228633. However, the therein used rather cAMP-sensitive vertebrate CNGA2 channel has an $EC_{50}$ of 39 $\mu$M (Dhallan et al., Nature 347, 184-187 (1990)). US 07166463 and US 07341836 disclose modified cyclic nucleotide-gated ion channels having an $EC_{50}$ of 14.5 $\mu$M for cAMP.

Recent findings indicate that odorant signal transduction in insects differs from mammalian signal transduction, since insect odorant receptors (ORs), which lack any sequence similarity to other GPCRs (Benton et al., PLoS Biol. 4, e20 (2006)), are heterodimers, composed of conventional odorant-sensitive ORs (e.g., Or22a), dimerised with a ubiquitously expressed chaperone protein (Neuhaus, et al., Nal. Neurosci. 8, 15-17 (2005), such as Or83b in *Drosophila* (Larsson et al., Neuron 43, 703-14 (2004)). Or83b has a structure akin to GPCRs, but has an inverted orientation in the plasma membrane (Benton et al., supra; Wistrand et al., Protein Sci. 15, 509-21 (2006); Lundin et al., FEBS Lett. 581, 5601-5604 (2007)) with an intracellular N-terminus and an extracellular C-terminus. It is assumed that Or83b is essential for olfaction, but the molecular mechanism by which it acts is unknown (Benton et al., supra).

Or83b is ubiquitously expressed and highly conserved among insect species (Neuhaus, et al., supra) and was therefore predicted as a target for insect control reagents (WO 03/016477; WO 2004/100971). However, it has never been described or suggested that Or83b protein without any additional OR protein, particularly an odorant-sensitive receptor protein, would be functionally active, let alone a useful mean for detecting a cyclic nucleotide level.

Therefore, one object of the present invention was to provide an alternative test system or a alternative method for detecting a cyclic nucleotide level, preferably wherein this test system or method should be sensitive and applicable in a high-throughput assay.

Surprisingly, the inventors found that heterologous expression of the Or83b in mammalian cells alone leads to functional ion channels which are directly activated by intracellular cAMP or cGMP. The high sensitivity of Or83b channels to nanomolar concentrations of cyclic nucleotides ($EC_{50}$ of 1.1 nM) improves cell-based assays reporting a change in cyclic nucleotide concentration. More specifically, the test system of the present invention allows high-throughput screening to identify ligands for all G protein coupled receptors (GPCRs) that affect cyclic nucleotide levels, based on transmembrane current measurements (as in population patch clamp or other automated patch clamp systems) or in monitoring the free intracellular calcium concentration.

Accordingly, a first aspect of the present invention relates to a test system for detecting a cyclic nucleotide, the test system comprising:

   i) a non-insect cell comprising an insect cyclic nucleotide-activated ion channel protein, capable of coupling to an insect odorant-sensitive receptor protein, or a functionally active derivative thereof, and
   ii) means for detecting the activity of the ion channel protein.

As detailed above the physiological, pathophysiological and biochemical role of different signal transduction pathways, particularly those including the members of the GPCR family, is not yet fully elucidated. Accordingly, tools for studying the function and control of such as GPCRs and substances modulating the same are required. In order to speed up identification and development of drugs and to minimize costs, sensitive but convenient tests are required e.g. for drug research. Preferred tests are applicable in a high-throughput assay.

The test system of the invention may be used in order to elucidate the function and activity of components involved in the signal transduction cascades including cyclic nucleotide signaling as initiated by many GPCRs. Particularly, mechanisms involving GPCRs that affect cAMP and/or cGMP levels can be studied by using the test system of the invention. Particularly the test system may be adapted to any of the methods of the invention as disclosed herein.

As a first component the test system of the invention encompasses a non-insect cell. The cell (i) may be any of the cells or cell lines disclosed in the context of the method of the invention and (ii) comprises an insect cyclic nucleotide-activated ion channel protein, as defined below. The test system further comprises means for detecting the activity of the ion channel protein which may be applied to the cell, as defined below. The test system may also encompass further components, which may be necessary in order to use the test system, e.g. in order to carry out any of the described methods or uses. Examples of those components include members of signal transduction pathways, either naturally occurring or artificial components, means for maintaining the cell, means for processing signals produced by the means for detecting the activity of the ion channel protein etc. (see also below).

Particularly, the test system is intended to be brought directly or indirectly into contact with an agent, in particular a (bio)chemical agent, e.g. in the form of a chemical compound library, or a physical stimulus. Simultaneously or subsequently, the activity of the ion channel protein upon the contacting may be detected. Methods and means of detecting the activity of the ion channel protein are detailed below. A change of the activity of the ion channel protein is indicative of a change in intracellular cyclic nucleotide level, or of the capability of the agent to modify an intracellular cyclic nucleotide level.

The term "detecting a cyclic nucleotide" relates to the detection of a single cyclic nucleotide or a mixture of cyclic nucleotides. A change in a singly nucleotide may be detected , e.g. if a signal cascade is involved which is coupled to a single cyclic nucleotide or if the insect cyclic nucleotide-activated ion channel protein or a functionally active derivative thereof is sensitive only to one type of cyclic nucleotide. In case of an cyclic nucleotide-unselective insect cyclic nucleotide-activated ion channel protein or a functionally active derivative thereof the total amount of cyclic nucleotides may be determined. In a preferred embodiment the insect cyclic nucleotide-activated ion channel protein or a functionally active derivative thereof is sensitive for cGMP or cAMP, particularly for only cAMP and cGMP.

A cyclic nucleotide is any nucleotide in which the phosphate group is bonded to two of the sugar's hydroxyl groups, forming a cyclical or ring structure, including cyclic AMP (cyclic AMP or 3'-5'-cyclic adenosine monophosphate), cyclic GMP (cyclic guanosine monophosphate), and cyclic ADP-ribose. For further details see also herein below.

As cAMP cyclic ADP-ribose, also known as cADPR, is a cyclic adenine nucleotide with two phosphate groups present on 5' OH of the adenosine, further connected to another ribose at the 5' position, which, in turn, closes the cycle by glycosidic bonding to N 1 of the same adenine base. It is produced from nicotinamide adenine dinucleotide (NAD$^+$) by ADP-ribosyl cyclases. cADPR is a cellular messenger for calcium signaling.

A "non-insect cell" as used herein relates to any cell or cell line other than an insect cell or insect cell line, i.e. to a cell or cell line which is not derived from an insect. The "non-insect cell" of the invention or its progenitor has been modified, particularly genetically modified (see below), to include the insect cyclic nucleotide-activated ion channel protein. Accordingly, it is also evident that the cell comprises one or more components of an insect cell, particularly the insect cyclic nucleotide-activated ion channel protein or derivative thereof, as defined herein. The cell may also comprise further components of an insect cell; however, the characteristics as a non-insect cell should be maintained. This could be judged e.g. by maintenance of the non-insect cell's morphology, when comparing the naturally occurring non-insect cell without insect components to the same non-insect cell having incorporated insect components.

Alternatively or additionally, the amount of insect genetic material incorporated into the non-insect cell could be limited. In accordance with this, the amount of insect genetic material included into the non-insect cell should preferably not exceed 20 % of the total amount of genetic material (in kb) in the resulting cell, more preferably not more than 10 %, still more preferably not more than 5 %, even more preferably not more the 4 %, 3 % or 2 %, and most preferably not more than 1 %.

In another preferred embodiment of the invention the number of insect genes and/or insect proteins in the non-insect cell may be limited. The upper limit could be at most 20 genes and/or proteins, preferably at most 10, still more preferably at most 9, 8, 7, 6, 5, 4, 3 or 2, most preferably one (i.e. only the insect cyclic nucleotide-activated ion channel protein and the gene coding the same). It should be noted that a gene and the respective encoded protein increase the number of insect genes and proteins only by 1. The skilled person will understand that the terms "insect genetic material", "insect gene(s)" and "insect protein(s)" relate to genetic material, gene(s) and protein(s), respectively, present only in a naturally-occurring insect or insect cell, but not in a naturally-occurring non-insect cell or organism, particularly not in naturally-occurring vertebrates, especially not in naturally-occurring mammals. "Naturally occurring" in the context of cell or organism is meant to refer to cells or organisms which are not the result of genetic engineering.

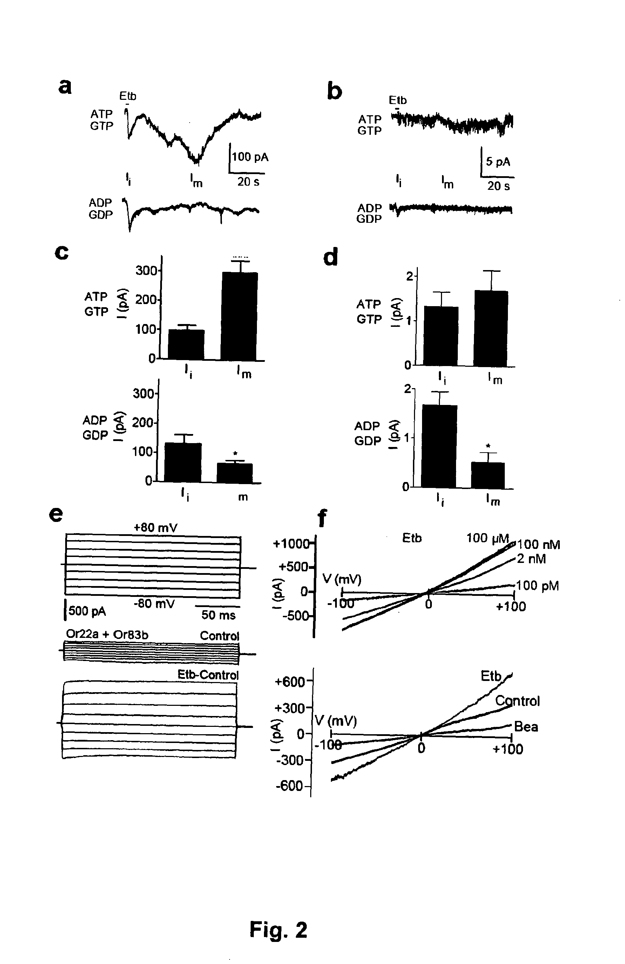

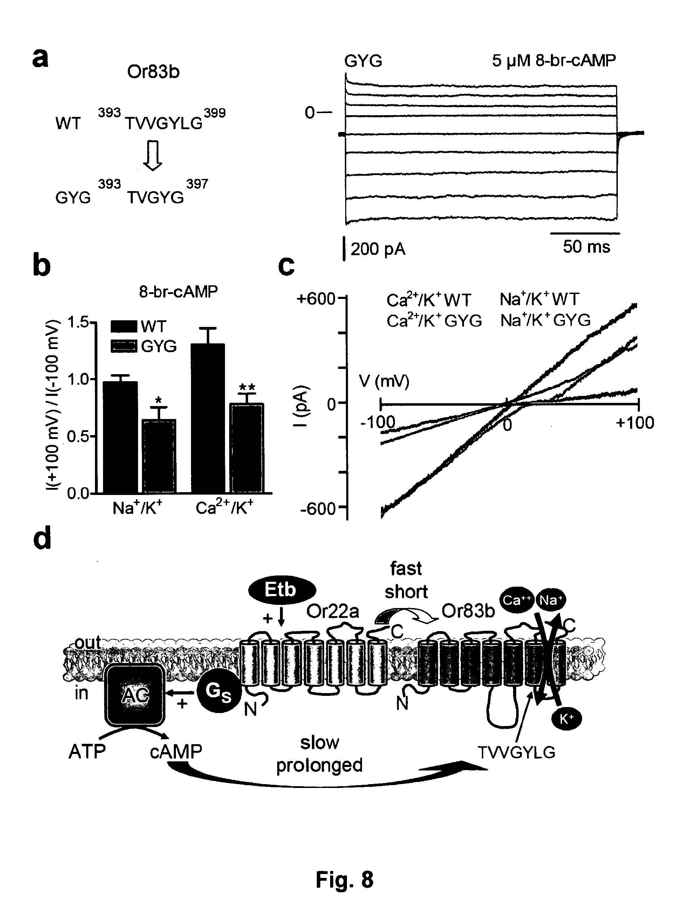

An "insect cyclic nucleotide-activated ion channel protein" as defined herein relates to an ion channel protein which is derived from an insect and which is activated in response to a cyclic nucleotide, especially cAMP and/or cGMP. It is noted that the cyclic nucleotide-activated ion channel protein is not sensitive to an odorant (i.e. odorant-insensitive). In insects odorant receptors are composed of a protein heterodimer consisting of an odorant-sensitive receptor protein and an odorant-insensitive channel protein (for illustration see also Fig. 8d, in which Or22a is the odorant-sensitive receptor protein and Or83b is the odorant-insensitive channel protein). In accordance with that the cyclic nucleotide-activated ion channel protein is capable of coupling to an insect odorant-sensitive receptor protein, i.e. the cyclic nucleotide-activated ion channel protein is capable of binding to the insect odorant-sensitive receptor protein. Upon binding of an odorant to the insect odorant-sensitive receptor protein, the cyclic nucleotide-activated ion channel protein is activated. "Not sensitive for an odorant" or "odorant-insensitive" in the present context means that the activity of the cyclic nucleotide-activated ion channel protein is not changed upon the direct interaction of cyclic nucleotide-activated ion channel protein with any odorant (see for e.g. Example 1, particularly Fig. 2). In contrast to the cyclic nucleotide-activated ion channel protein, the odorant-sensitive receptor protein is sensitive to odorant, i.e. it is activated upon binding of the odorant to the receptor protein.

An ion channel or ion channel protein regulates the flux of one or more types of ions across the membrane in cells. It is an integral membrane protein forming a water-filled pore through the cell membrane. While some channels permit the passage of ions based solely on charge, the archetypal channel pore is just one or two atoms wide at its narrowest point. It conducts a specific species of ion, such as sodium or potassium, and conveys them through the membrane. In the cyclic nucleotide-activated ion channel protein, passage through the pore is governed by a "gate", which is be opened at a particular concentration of a cyclic nucleotide-activated and closed at lower concentrations of the cyclic nucleotide.

Accordingly, activated in response to a cyclic nucleotide, including cAMP and/or cGMP, is intended to mean that the ion channel protein is opened to allow for passage of the respective ion or ions. As detailed above, the ion channel protein may be quite selective for a particular ion or it may be less selective. For example it may be selective for positively charged ions including K$^+$, Na$^+$, Zn$^{2+}$ and Ca$^{2+}$, particularly K$^+$, Na$^+$, and Ca$^{2+}$, especially Ca$^{2+}$. Alternatively, the channel allows only for passage of one sort of cation selected from the group consisting of K$^+$, Na$^+$, Zn$^{2+}$ and Ca$^{2+}$.

One example of such an insect nucleotide-activated ion channel protein is the odorant receptor 83b (Or83b) from insect, particularly from Or83b *Drosophila melanogaster* (derivable from protein databases such as NCBI (National Center for Biotechnology Information) under accession numbers AAT71306 or Q9VNB5 or AY567998), which is also referred to DOr83b. DOr83b is ubiquitously expressed in olfactory neurons from *Drosophila melanogaster*. Or83b proteins constitute a class of ion channel proteins highly conserved among insect species. As detailed above, it forms heterodimeric complexes with other odorant-receptor proteins, namely odorant-sensitive receptor proteins. Receptors like Or83b show a typical seven transmembrane alpha helical structure, with an intracellular N-terminus and an extracellular C-terminus. Homologues of *Drosophila melanogaster* Or83b are, for example, further disclosed for *Arropheles gambiae* (accession number AY843205), *Ceratitis capitata* (accession number AY843206), *Helicoverpa zea* (accession number AY843204), and *Helicoverpa assulta* (accession number EU057178). The term "homologues of Or83b" relates to naturally occurring variants of Or83b, i.e. proteins having the same function (i.e. cyclic nucleotide-activated ion channel function and capability of coupling to an odorant-sensitive receptor protein), which are derived from other insect species and/or which are splice variants. Further homologues of Or83 may be identified by a sequence alignment, e.g. by using the BLAST gene library alignment tool of the National Center for Biotechnology Information (NCBI) with a given Or83b sequence as a template. Functional homologues may also be identified by screening for structure homology in a protein database such as the Expert Protein Analysis System (ExPASy) proteomics server of the Swiss Institute of Bioinformatics (SIB). Homologues may further be identified by screening DNA or cDNA libraries derived from the species in question by using labelled probes typically complementary to a conserved specific sequence region of Or83b. These probes are incubated with the DNA or cDNA library under conditions, that allow binding of the probe to its homologous complementary sequence, but no other unspecific DNA sequence. Alternatively a polymerase chain reaction may be used to specifically amplify and isolate the homologous gene from a gene or DNA library, or, e.g. by using an anchored PCR, the cDNA from a cDNA library.

In a further embodiment of the present invention a functionally active derivative of a naturally occurring cyclic nucleotide-activated ion channel protein (e.g. Or83b or homologue thereof) may be part of the test system of the present invention. In contrast to a homologue, a functionally active derivative relates to an artificial cyclic nucleotide-activated ion channel protein having been obtained by genetic engineering. A "derivative" of an insect cyclic nucleotide-activated ion channel protein according to the invention is derived from the original, i.e. wild-type, amino acid sequence in question, thus an artificially modified version of the original sequence. The derivative is functionally active, which means that the derivative is still activated by a cyclic nucleotide and maintains its channel function. However, the derivative need not be capable of coupling to an odorant-sensitive receptor protein. The biological activity shown by a derivative can be the full activity when compared to the wild-type ion channel protein under identical conditions, or can be less than full activity, e.g. 10 %, 20 %, 30 %, 40 %, 50 %, 55 %, 60 %, 65 %, 70 %, 75 %, 80 %, 85 %, 90 %, 95 %, or 99 % of the activity of the wild-type polypeptide when compared under identical conditions. The biological activity of an insect cyclic nucleotide-activated ion channel protein can be evaluated by a skilled person e.g. by comparing the ion flux through the activated derivative ion channel protein to the corresponding activated wild-type ion channel protein. For example, the activity may be determined as detailed in Example 1 in connection with the experiment shown in Fig. 8.

In one embodiment of the present invention the derivative of an insect cyclic nucleotide-activated ion channel protein has e.g. 50 %, 55 %, 60 %, 65 %, 70 %, 75 %, 80 %, 85 %, 90 %, 95 %, or 100 % amino acid sequence identity to the original or wild-type amino acid sequence, when both amino acid sequences are aligned using conventional alignment programs. Preferably, a derivative of an insect cyclic nucleotide-activated ion channel protein according to the invention has 50 %, 55 %, 60 %, 65 %, 70 %, 75 %, 80 %, 85 %, 90 %, 95 %, or 100 % sequence identity to any of the sequences disclosed under accession numbers AAT71306 or Q9VNB5, or to the amino acid sequences encoded by the nucleotide sequences AY567998, AY843205, AY843206, AY843204, or EU057178.

Generally, amino acid sequence has "at least x % identity" with another amino acid sequence or any of the sequences given above if, when the sequence identity between those to aligned sequences is at least x %. Such an alignment can be performed using for example publicly available computer homology programs such as the "BLAST" program provided at the NCBI homepage at http://www.ncbi.nlm.nih.gov/blast/blast.cgi, using the default settings provided therein. Further methods of calculating sequence identity percentages of sets of nucleic acid sequences are known in the art.

The "derivative" may be obtained by one or more amino acid substitution(s), addition(s) or deletion(s) or any combination thereof, as long as the functionality of the derivative is maintained (see also above). The skilled person is capable of preparing protein variants, e.g. by means of genetic engineering (see also below) using e.g. restriction enzymes or as described in Example 1.

In order to maintain biological activity, the derivative insect cyclic nucleotide-activated ion channel protein may preferably comprise conservative substitutions or semi-conservative substitutions. Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

Typical semi-conservative and conservative substitutions are:

| Amino acid | Conservative substitution | Semi-conservative substitution |
| --- | --- | --- |
| A | G; S; T | N; V; C |
| C | A; V; L | M; I; F; G |
| D | E; N; Q | A; S; T; K; R; H |
| E | D; Q; N | A; S; T; K; R; H |
| F | W; Y; L; M; H | I; V; A |
| G | A | S; N; T; D; E; N; Q |
| H | Y; F; K; R | L; M; A |
| I | V; L; M; A | F; Y; W; G |
| K | R; H | D; E; N; Q; S; T; A |
| L | M;I;V;A | F; Y; W; H; C |
| M | L; I; V; A | F; Y; W; C; |
| N | Q | D; E; S; T; A; G; K; R |
| P | V; I | L; A; M; W; Y; S; T; C; F |
| Q | N | D; E; A; S; T; L; M; K; R |
| R | K; H | N; Q; S; T; D; E; A |
| S | A; T; G; N | D; E; R; K |
| T | A; S; G; N; V | D; E; R; K; I |
| V | A; L; I | M; T; C; N |
| W | F;Y;H | L; M; I; V; C |
| Y | F; W; H | L; M; I; V; C |

Furthermore, the skilled person will appreciate that glycines at sterically demanding positions should not be substituted and that proline should not be introduced into parts of the protein which have an alpha-helical or a beta-sheet structure.

Alternatively, a derivative may also be a chimeric insect cyclic nucleotide-activated ion channel protein comprising domains or segments of two or more different ion cyclic nucleotide-activated ion channel proteins. The chimeric derivative may also encompass one or more segments with mutations (e.g. substitutions, additions and/or deletions) which may alter the activity and sensitivity of the derivative insect cyclic nucleotide-activated ion channel protein.

In a still further alternative, the derivative may be a fusion protein comprising an insect cyclic nucleotide-activated ion channel protein or functionally active derivative thereof and a further protein component. The further component may be a tag (e.g. for purification) a marker (e.g. for localization), an inoperable part (e.g. resulting from genetic manipulation) etc. Exemplary fusion proteins are given in the Examples.

In a preferred embodiment the derivative is an Or83b protein with mutations in the glycosylation acceptor sites, such as disclosed in Lundin et al., FEBS Letters, 581: 5601-5604 (2007). In another preferred embodiment the derivative is an Or83b protein with a mutation in the putative pore region, preferably a deletion of the wild-type Or83b amino acids $V^{394}$ or $V^{395}$, and $L^{398}$, thereby replacing the wild-type TVVGYLG (SEQ ID NO:1) motif with TVGYG (SEQ ID NO:2) (see Example 1 and Fig. 8).

As a second component, the test system of the invention encompasses means for detecting the activity of the ion channel protein. As detailed above, activity of the ion channel protein correlates with ion flux through the channel. Accordingly, the means for detecting the activity of the ion channel protein are typically means for detecting an ion, ion flux or current. Accordingly, the means of detecting the activity of the ion channel protein may comprise a marker, preferably a fluorescent marker, or means for detecting an ion, ion flux or current, preferably a microelectrode, a patch-electrode, or a high-throughput patch-electrode.

Fluorescence is an optical phenomenon, wherein the molecular absorption of a photon triggers the emission of another photon with a longer wavelength. The energy difference between the absorbed and emitted photons ends up as molecular vibrations or heat. Usually, the absorbed photon is in the ultraviolet range, and the emitted light is in the visible range, but this depends on the absorbance curve and Stokes shift of the particular fluorophore.

In one embodiment of the invention a fluorescent dye or marker or label is used in order to detect the activity of the ion channel protein. The fluorescent dye comprises a fluorophore. A fluorophore, in analogy to a chromophore, is a component of a molecule which causes a molecule to be fluorescent. It is a functional group in a molecule which will absorb energy of a specific wavelength and re-emit energy at a different (but equally specific) wavelength. The amount and wavelength of the emitted energy depend on both the fluorophore and the chemical environment of the fluorophore.

In the context of the present invention the fluorescent marker is sensitive for the effect of the activation of the insect cyclic-nucleotide-activated ion channel protein. The effect may be a change in the ion flux through the ion channel protein, particularly a change in the intracellular concentration of the ion transported by the ion channel protein. The ion may be, for example, $K^+$, $Na^+$, $Zn^{2+}$ or $Ca^{2+}$. Calcium ions may be detected by using aequorin (the first photoprotein to be isolated), calmodulin labeled with Alexa-488; Fura-2, Indo-1, Fluo-3, Fluo-4 (Molecular Probes); Rhod-2 or photina (Axxam SpA, Milan, Italy). For the detection of sodium and potassium ions ionophore X and ionophore BME-44 may be employed. Zinc ions may be detected by Fluozin-3. A suitable device for measuring fluorescent markers, particularly in high-through put assays, is for example FLUOstar 97 fluorometer multi-well plate reader (BMG LabTechnologies, Inc, Durham, NC).

Classical electrophysiological techniques involve placing electrodes into various preparations of biological tissue. The principal types of electrodes are: 1) simple solid conductors, such as discs and needles (singles or arrays), 2) tracings on printed circuit boards, and 3) hollow tubes filled with an electrolyte, such as glass pippettes. The principal preparations include cells, tissues or organisms. Intracellular recording involves measuring voltage and/or current across the membrane of a cell. To make an intracellular recording, the tip of a fine (sharp) microelectrode is inserted inside the cell, so that the membrane potential can be measured. The voltage clamp uses a negative feedback mechanism and allows an experimenter to "clamp" the cell potential at a chosen value. This makes it possible to measure how much ionic current crosses a cell's membrane at any given voltage. The current clamp technique records the membrane potential by injecting current into a cell through the recording electrode. Unlike in the voltage clamp mode, where the membrane potential is held at a level determined by the experimenter, in "current clamp" mode the membrane potential is free to vary, and the amplifier records whatever voltage the cell generates on its own or as a result of stimulation. This technique is used to study how a cell responds when electrical current enters a cell; this is important for instance for understanding how neurons respond to neurotransmitters that act by opening membrane ion channels.

Alternatively or additionally, ion-sensitive microelectrodes (including an ion-selective ionophore such as the sodium-ionophore Bis(12-crown-4), the potassium-selective ionophor Bis(benzo-15-crown-5), the calcium-selective ionophore HDOPP-Ca) or ion sensitive enzymes (such as potassium-dependent urea amidolyase, pyruvate kinase (U.S. Pat. Nos. 5,501,958 and 5,334,507) or glycerol dehydrogenase (U.S. Pat. No. 5,719,036)) may be used for the detection of a particular ion flux across cell membranes. Preferably, the method is adapted for high-through put screening. In this method a large number of compounds are screened against a component of a signal transduction pathway involving change in a cyclic nucleotide level, such as a GPCR in question, using the test system in whole-cell assays. Typically, these screenings are carried out in 96 well plates using automated, robotic station based technologies or in high-throughput assay formats, as defined below.

In a further embodiment of the invention, the patch clamp technique is used in order to detect the activity of the ion channel protein. Patch clamp technique allows the study of individual ion channels in cells. In classical patch clamp technique, the electrode used is a glass pipette, but planar patch clamp uses a flat surface punctured with tiny holes. The cell-attached patch clamp uses a micropipette attached to the cell membrane to allow recording from a single ion channel. Patch clamp traditionally uses a glass pipette, with an open tip diameter of about one micrometre, and is made such that the tip forms a smooth surfaced circle, rather than a sharp point. This style of electrode is known as a "patch clamp electrode" and is distinct from the "sharp microelectrode" used to impale cells in traditional intracellular recordings (see above). The interior of the pipette is filled with different solutions depending on the specific technique or variation used (see following). For example, with whole cell recordings, a solution that approximates the intracellular fluid is used. A metal electrode in contact with this solution conducts the electrical changes to a voltage clamp amplifier. The researcher can change the composition of this solution or add drugs to study the ion channels under different conditions. The patch clamp electrode is pressed against a cell membrane and suction is applied to the inside of the electrode to pull the cell's membrane inside the tip of the electrode. The suction causes the cell to form a tight seal with the electrode. Unlike traditional voltage clamp recordings, the patch clamp recording uses a single electrode to voltage clamp a cell. This allows a researcher to keep the voltage constant while observing changes in current. Alternately, the cell can be current clamped, keeping current constant while observing changes in membrane voltage.

Several variations of the basic technique can be applied, depending on what the researcher wants to study. The inside-out and outside-out techniques are called "excised patch" techniques, because the patch is excised (removed) from the main body of the cell. Cell-attached and both excised patch techniques are used to study the behavior of ion channels on the section of membrane attached to the electrode, while whole-cell patch and perforated patch allow the researcher to study the electrical behavior of the entire cell.

In the cell-attached patch the electrode remains sealed to the patch of membrane. This allows for the recording of currents through single ion channels in that patch of membrane. For channels that are activated through the action of drug molecules, the drug of choice is usually included in the pipette solution.

In the "inside-out" patch the electrode is quickly withdrawn from the cell after the seal is formed, thus ripping the patch of membrane off the cell, leaving the patch of membrane attached to the electrode exposing the intracellular surface of the membrane to the external media. This is useful when an experimenter wishes to manipulate the environment affecting the inside of ion channels. For example, channels that are activated by intracellular ligands like cAMP and/or cGMP can then be studied through a range of ligand concentrations.

In the whole-cell recording or whole-cell patch the electrode is left in place, but more suction is applied to rupture the portion of the cell's membrane that is inside the electrode, thus providing access to the intracellular space of the cell. The advantage of whole-cell patch clamp recording over sharp microelectrode recording is that the larger opening at the tip of the patch clamp electrode provides lower resistance and thus better electrical access to the inside of the cell. A disadvantage of this technique is that the volume of the electrode is larger than the cell, so the soluble contents of the cell's interior will slowly be replaced by the contents of the electrode. Whole-cell recordings involve recording currents through multiple channels at once.

In the "outside-out" patch the electrode can be slowly withdrawn from the cell after the aforementioned whole cell patch is formed, allowing a bulb of membrane to bleb out from the cell. When the electrode is pulled far enough away, this bleb will detach from the cell and reform as a ball of membrane on the end of the electrode, with the outside of the membrane being the surface of the ball. Outside-out patching gives the experimenter the opportunity to examine the properties of an ion channel when it is protected from the outside environment, but not in contact with its usual environment. In this conformation, the experimenter can perfuse the same patch with different solutions, and if the channel is activated from the extracellular face, a dose-response curve can then be studied. Single channel recordings are possible in this conformation if the bleb of membrane is small enough.

The perforated patch is a variation of whole-cell recording, in which the experimenter forms the seal, then adds a new solution to the electrode containing small amounts of an antibiotic, such as Amphothericin-B or Gramicidin into the electrode solution to punch small perforations on the bit of membrane attached to the electrode. This has the advantage of reducing the dialysis of the cell that occurs in whole cell recordings.

Planar patch clamp, i.e. a method involving a high-throughput patch electrode, is a novel method developed for high throughput electrophysiology. Instead of positioning a pipette on an adherent cell, cell suspension is pipetted on a chip containing a microstructured aperture. A single cell is then positioned on the hole by suction and a tight connection (Gigaseal) is formed. The planar geometry offers a variety of advantages compared to the classical experiment: - it allows for integration of microfluidics, which enables automatic compound application for ion channel screening. - the system is accessible for optical or scanning probe techniques - perfusion of the intracellular side can be performed.

Furthermore, the Bioelectric Recognition Assay (BERA) is a novel method for measuring changes in the membrane potential of cells immobilized in a gel matrix. Apart from the increased stability of the electrode-cell interface, immobilization preserves the viability and physiological functions of the cells. BERA is primary used in biosensor applications in order to assay analytes which can interact with the immobilized cells by changing the cell membrane potential. In this way, when a positive sample is added to the sensor, a characteristic, 'signature-like' change in electrical potential occurs. A recent advance in the evolution of the BERA technology was the development of a technique called Molecular Identification through Membrane Engineering (MIME). This technique allows for building cells with absolutely defined specificity against virtually any molecule of interest, by embedding thousand of artificial receptors into the cell membrane.

However, the skilled person will understand that the above techniques may employ the method of detecting a change of an intracellular cyclic nucleotide level, or the method of identifying an agent capable of modifying an intracellular cyclic nucleotide level on cell membrane fragments having been obtained from the non-insect cell of the present invention (e.g. the outside-out patch or inside-out patch methods). Particularly, any of the methods of the invention starts with a whole non-insect cell of the invention, for example wherein the patch-electrode or patch-pipette is attached to the cell of the test system in order to obtain the above patched membrane fragment including the insect cyclic nucleotide-activated ion channel protein. In accordance with this, the agent or condition may be applied to the patched membrane fragment. If a cell membrane fragment is used, the feature "intracellular" in the context of the "method of detecting a change of an intracellular cyclic nucleotide level" or "method of identifying an agent capable of modifying an intracellular cyclic nucleotide level" relates to the orientation of the insect cyclic nucleotide-activated ion channel in the membrane, i.e. the side of the membrane fragments which would be intracellular in a whole cell.

In a preferred embodiment of the invention the non-insect cell does not comprise an insect odorant-sensitive receptor protein.

The non-insect cell may be a vertebrate cell or cell line, preferably a mammalian cell or cell line, more preferably a mouse, rat, primate or human cell or cell line. Optionally, the cell may be part of a tissue; however, the method of the invention is an *ex vivo* method, unless the respective patent law or jurisdiction allows also for *in vivo* methods. In a preferred embodiment of the invention the cell is from a cell line (many of which are well characterized and provide for constant conditions and convenient handling), particularly a mammalian cell line, more particularly a mouse, rat, primate or human cell line, especially human HEK 293 cell line. Examples of suitable cell lines include but are not limited to HEK 293, CHO, COS-1, COS-7, HEK, HeLa, Jurkat, Ramos, U-937 or Raji. Most preferably the cell line is a HEK293 cell line, especially the one of the HEK293 cell line marketed by BD under the trademark ACT:One™, which is adapted for receptor research. In general, also insect cells or cell lines would be suitable. Suitable cell lines are commercially available from culture collection such as the ATCC or from other commercial suppliers and optionally express an additional component of the test system as defined below. Additionally, the non-insect cell may be a yeast cell, such as a *Saccharomyces cerevisiae* cell, or an artificial cell, such as a liposome, comprising necessary signal translation pathway components. Other suitable cells are those known to the one of skill in the art. It is noted that those cells or cell lines, particularly totipotent human embryonic stem cell, are excluded, which are not subject to patentability under the respective patent law or jurisdiction. However, the cell or cell lines may need to be adapted to include the insect cyclic nucleotide-activated ion channel protein, capable of coupling to an insect odorant-sensitive receptor protein, or a functionally active derivative thereof, as defined above.

The cell may be isolated (and genetically modified), maintained and cultured at an appropriate temperature and gas mixture (typically, 37°C, 5% $CO_2$), optionally in a cell incubator as known to the skilled person. Culture conditions may vary for each cell type, and variation of conditions for a particular cell type can result in different phenotypes. Aside from temperature and gas mixture, the most commonly varied factor in cell culture systems is the growth medium. Recipes for growth media can vary in pH, glucose concentration, growth factor and the presence of other nutrient components among others. Growth media are either commercially available, or can be prepared according to compositions, which are obtainable from the American Tissue Culture Collection (ATCC). Growth factors used for supplement media are often derived from animal blood such as calf serum. Additionally, antibiotics may be added to the growth media. Amongst the common manipulations carried out on culture cells are media changes and passaging cells.

In accordance with the present invention, the non-insect cell may comprise component i), as well as component ii) of the test system of the invention, for example if a fluorescent marker is expressed in the cell. Genetically modified cells may be obtained by inserting the full-length coding sequence of the insect cyclic nucleotide-activated ion channel protein and optionally further components as defined below, as known to the skilled person. The skilled person in the art knows how to derive a nucleic acid sequence coding for a protein and how to isolate or produce such a nucleic acid sequence using standard techniques of molecular biology. This can be accomplished, for example, by the use and combination of existing sequences using restriction enzymes.

The nucleic acid coding for a protein (used for the present invention) may be a naturally occurring gene coding for an insect cyclic-nucleotide activated ion channel protein or a functionally active derivative thereof, means for detecting the activity of the channel protein or a further component involved in the change of cyclic nucleotide level in a cell, as defined below. The isolation of nucleic acids coding for naturally occurring proteins or a part thereof is well known to the skilled person and may include the isolation using suitable probes and separation methods, or the nucleic acid may be derived from a commercial supplier. If a nucleic acid is used which is not comprised by a naturally occurring gene (e.g., nucleic acid coding for a fusion protein, mutated or truncated forms) the respective nucleic acid coding for the fusion protein may be produced in accordance with standard procedures including well-known methods of genetic engineering.

The nucleic acid may be combined with further elements, e.g., a promoter and a transcription start and stop signal and a translation start and stop signal and a polyadenylation signal in order to provide for expression of the sequence of the protein of the invention. The promoter can be inducible or constitutive, general or cell specific, nuclear or cytoplasmic specific promoter. Selection of promoters, vectors and other elements is a matter of routine design within the level of ordinary skill in the art. Many such elements are described in the literature and are available through commercial suppliers.

The resulting nucleic acid sequence may be introduced into cells e.g. using a virus as a carrier or by transfection including e.g. by chemical transfectants (such as Lipofectamine, Fugene, etc.), electroporation, calcium phosphate co-precipitation and direct diffusion of DNA. A method for transfecting a cell is detailed in Example 1 or 2 and may be adapted to the respective recipient cell. Transfection with DNA yields stable cells or cell lines, if the transfected DNA is integrated into the genome, or unstable (transient) cells or cell lines, wherein the transfected DNA exists in an extrachromosomal form. Furthermore, stable cell lines can be obtained by using episomal replicating plasmids, which means that the inheritance of the extrachromosomal plasmid is controlled by control elements that are integrated into the cell genome. In general, the selection of a suitable vector or plasmid depends on the intended host cell.

In general, a cell line may be transfected by any of the means mentioned above, wherein the transgene may be operatively linked to a selectable marker. Following transfection cells are grown e.g. for some days in enriched media and then switched to selective media. Transfected cells exhibit resistance to the selection and are able to grow, whereas non-transfected cells die in general. Examples for selective markers include puromycin, zeocin, neomycin (neo) and hygromycin B, which confer resistance to puromycin, zeocin, aminoglycoside G-418 and hygromycin, respectively, and/or any of the selective markers used in the Examples. However, other selection methods known to the skilled person may be also suitable.

In a preferred embodiment of the present invention the insect cyclic nucleotide-activated ion channel protein is insect Or83b or a functionally active homologue, or a functionally active derivative thereof, particularly wherein the insect cyclic mucleotide-activated channel protein is *Drosophila melanogaster* Or83b or a functionally active derivative thereof. The insect cyclic nucleotide-activated channel may be Or83b, as defined above, preferably Or83b derived from an insect of the order *Diptera*, more preferably from an insect of the family *Drosophilidae,* even more preferably from an insect of the subfamily *Drosophilinae*, most preferably from an insect of the genus *Drosophila* and even most preferably from an insect of the species *Drosophila melanogaster*, as for example disclosed in protein databases such as NCBI (National Center for Biotechnology Information) under accession numbers AAT71306 or Q9VNB5 or AY567998. The insect cyclic nucleotide-activated ion channel protein may also be a functionally active homologue or a functionally active derivative, as defined above, of Or83b.

In a preferred embodiment of the present invention the cyclic nucleotide is cAMP or cGMP.

Cyclic adenosine monophosphate (cAMP, cyclic AMP or 3'-5'-cyclic adenosine monophosphate) is synthesised from ATP by adenylyl cyclase. Some research has suggested that a deregulation of cAMP pathways and an aberrant activation of cAMP-controlled genes is linked to the growth of some cancers. Recent research may indicate that cAMP affects the function of higher order thinking in the prefrontal cortex through its regulation of ion channels called hyperpolarization-activated cyclic nucleotide-gated channels (HCN). This research is of interest in understanding the degradation of higher cognitive function in Attention-Deficit Hyperactivity Disorder (ADHD) and aging.

At present, nine isoforms of adenylyl cyclase have been identified. All adenylyl cyclase isoforms are stimulated by $G_s$ proteins, and by forskolin, as well as other class-specific substrates. Isoforms I, III and VIII are also stimulated by $Ca^{2+}$/calmodulin, whereas isoforms V and VI are inhibited by $Ca^{2+}$ in a calmodulin-independent manner.

Cyclic guanosine monophosphate (cGMP) is derived from guanosine triphosphate (GTP). cGMP acts as a second messenger much like cyclic AMP, most notably by activating intracellular protein kinases. cGMP synthesis is catalyzed by guanylyl cyclase which converts GTP to cGMP. cGMP is a common regulator of ion channel conductance, glycogenolysis, as well as cellular apoptosis and also relaxes smooth muscle tissues. In blood vessels, relaxation of vascular smooth muscles leads to vasodilation and increased blood flow. As another example, cGMP is a secondary messenger in phototransduction in the eye. In the photoreceptors of the mammalian eye, the presence of light activates phosphodiesterase, which degrades cGMP. The sodium ion channels in photoreceptors are cGMP-gated, so degradation of cGMP causes sodium channels to close, which leads to the hyperpolarization of the photoreceptor's plasma membrane and ultimately to visual information being sent to the brain.

Membrane-bound guanylyl cyclase is activated by peptide hormones such as the atrial natriuretic factors including atrial natriuretic factor or heat-stable enterotoxins, while soluble GC is typically activated by nitric oxide to stimulate cGMP synthesis.

In a preferred embodiment of the invention the means of detecting the activity of the ion channel comprise a marker, preferably a fluorescent marker, or means for detecting a current, an ion flux or ion, preferably a microelectrode, a patch-electrode, or a high-throughput patch-electrode. For further details see above.

In a preferred embodiment of the invention the non-insect cell further comprises one or more additional component(s) involved in the change of cyclic nucleotide level in a cell, preferably wherein the one or more additional component is selected from a group consisting of:

- a G protein-coupled receptor (GPCR), preferably wherein the GPCR is capable of coupling to a $G_s$ protein or to a $G_i$ protein, more preferably wherein the GPCR is capable of coupling to a $G_s$ protein;
- a G protein or a G protein-chimera capable of coupling to adenylyl or guanylyl cyclase, preferably wherein the G protein is a $G_s$ protein or a $G_i$ protein, more preferably wherein the G protein is a $G_s$ protein;
- an adenylyl cyclase; and
- a guanylyl cyclase.

There is a plurality of other additional components known in the art that are involved in cyclic nucleotide signaling pathways, and these may be employed in the test system of the invention. These may be any component which directly or indirectly influences an intracellular cyclic nucleotide level, particularly GPCR and G proteins and effector such as adenylyl cyclase and guanylyl cyclase.

The family of G protein coupled receptors is a large superfamily of integral membrane proteins. Currently there are about 2,000 G protein coupled receptor sequences available from public databases. Some of the receptors and their endogenous ligands as well as exogenous agonists and antagonists have been known for a long time (e.g. the $\beta_2$-adrenergic receptor). Others are so-called orphan G protein coupled receptors, since they do not bind to any known endogenous ligand.

G protein coupled receptors (also known as 7-transmembrane, heptahelical or serpentine receptors) exhibit amino acid sequence as well as structural similarities. They are composed of a single polypeptide chain which spans the cell membrane seven times and consist of an extracellular amino terminus, seven predominantly hydrophobic α-

EP 2 108 954 A1

helical domains (of about 20-30 amino acids) spanning the cell membrane, approximately 20 well-conserved amino acids and a cytoplasmic carboxy terminus.

Examples of GPCR are without limitation beta1-adrenoreceptor, beta2-adrenoreceptor, adenosine A2b receptor, prostaglandin E2 receptor, adenosine A1 receptor, adenosine A3 receptor, cannabinoid receptor 1, cannabinoid receptor 2, dopamine D2 receptor, endothelial differentiation, sphingolipid G-protein-coupled receptor 1 (EDG1), metabotropic 7 receptor, metabotropic 8 receptor, melanin concentrating hormone receptor 1 (MCHR1), neuropeptide Y1 receptor (NPY1R), neuropeptide Y2 receptor (NPY2R), somatostatin receptor 5 (SSTR5), adenosine A2a receptor, adenylate cyclase activating polypeptide 1 (pituitary) receptor type 1 (ADCYAP1R1), arginine vasopressin receptor 2 (AVPR2), calcitonin receptor (CALCR), calcitonin-like receptor (CALCRL), corticotropin releasing hormone receptor 1 (CRHR1), corticotropin releasing hormone receptor 2 (CRHR2), dopamine receptor D1 (DRD1), dopamine receptor 5 (DRD5), follicle stimulating hormone receptor (FSHR), glucagon receptor (GCGR), glucagon-like peptide 1 receptor (GLP1R), glucagon-like peptide 2 receptor (GLP2R), gastric inhibitor peptide receptor (GIPR), melanocortin 1 receptor (MC1R), melanocortin 3 receptor (MC3R), melanocortin 4 receptor (MC4R), melanocortin 5 receptor (MC5R), parathyroid hormone receptor 1 (PTHR1), parathyroid hormone receptor 2 (PTHR2), prostaglandin E4 receptor (PTGER4), prostaglandin D2 receptor (PTGDR2), prostaglandin I2 receptor (PTGIR), secretin receptor (SCTR), serotonin receptor 6 (HTR6), serotonin receptor 7 (HTR7B), thyroid stimulating hormone receptor (TSHR), vasoactive intestinal peptide receptor 1 (VIPR1) and vasoactive intestinal peptide receptor 2 (VIPR2).

The binding of an agonist to its receptor mediates a change in receptor conformation whereby the receptor is transferred to its active form. The receptor in its active form activates the G protein and mediates the exchange of GTP for GDP. Antagonists (blockers) stabilise the receptor in its inactive form thus blocking the prevailing downstream signal transduction pathway.

G protein coupled receptors transduce the signal via heterotrimeric guanine nucleotide-binding proteins (G proteins). Heterotrimeric G proteins consist of an $\alpha$ (39-42 kDa), a $\beta$ (35-36 kDa), and a $\gamma$ subunit (7-10 kDa). The $\alpha$ subunit harbours a binding site for GTP/GDP. G proteins are naturally occurring on the cytoplasmic side of the plasma membrane.

Binding of an extracellular ligand leads to a conformational change in the receptor protein that allows it to make contact with a guanine-nucleotide binding protein (G protein). The activated G protein coupled receptor alters the confirmation of the $\alpha$ subunit and enhances the exchange of GTP for GDP. In the inactive state the $\beta\gamma$ dimer is bound to the $\alpha$ subunit. Upon the exchange, the $\beta\gamma$ dimer dissociates from the $\alpha$ subunit. Both the activated $\alpha$ subunit and the $\beta\gamma$ dimer can influence intracellular effector proteins. The $\alpha$ subunit then catalyses the hydrolysis of GTP into GDP and $P_i$, which causes the inactivation of the $\alpha$ subunit and subsequently the binding of the $\beta\gamma$ dimer to the $\alpha$ subunit.

Presently, the G protein $\alpha$ subunit family is grouped in four different classes, i.e. $G_{\alpha s}$, $G_{\alpha i}$, $G_{\alpha q}$ and $G_{\alpha 12}$. $G_{\alpha s}$ and $G_{\alpha i}$ classes stimulate or inhibit adenylyl cyclase activity, respectively. The $G_{\alpha q}$ and $G_{\alpha 12}$ class stimulate phospholipase C.

In general, G protein coupled receptors activate specifically a particular G protein $\alpha$ subunit family, which leads to the activation or inactivation of a particular signal transduction pathway. Many GPCR are known to stimulate or inhibit adenylyl cyclase after activation of the receptor. Examples of those GPCR (which may be used in the present invention) and their effects include without limitation:

| Receptor | Effect mediated by agonist |
|---|---|
| $M_1$ (muscarinic receptor) | Elevation of cAMP level |
| $M_2$ (muscarinic receptor) | Inhibition of adenylyl cyclase |
| $M_3$ (muscarinic receptor) | Elevation of cAMP level |
| $M_4$ (muscarinic receptor) | Inhibition adenylyl cyclase Stimulation of adenylyl cyclase |
| $M_5$ (muscarinic receptor) | Elevation of cAMP level |
| $A_1$ (adenosine receptor) | Inhibition of adenylyl cyclase |
| $A_2$ (adenosine receptor) | Stimulation of adenylyl cyclase |
| $A_3$ (adenosine receptor) | Inhibition of adenylyl cyclase |
| $\alpha$ ($\alpha_1$-adrenoceptor) | Activation of $G_{q/11}$ |
| $\alpha_2$ ($\alpha_2$-adrenoceptor) | Inhibition of adenylyl cyclase |
| $\beta_1$ ($\beta_1$-adrenoceptor) | Stimulation of adenylyl cyclase |
| $\beta_2$ ($\beta_2$-adrenoceptor) | Stimulation of adenylyl cyclase |

(continued)

| Receptor | Effect mediated by agonist |
|---|---|
| $\beta_3$ ($\beta_3$-adrenoceptor) | Stimulation of adenylyl cyclase |
| $\beta_4$ ($\beta_4$-adrenoceptor) | Stimulation of adenylyl cyclase |
| $AT_1$ (angiotensin recepor) | Decrease in cAMP |
| $B_1$ (bradykinin receptor) | Activation of $G_{q/11}$ |
| $B_2$ (bradykinin receptor) | Activation of $G_{q/11}$ |
| $CB_1$ (cannabinoid receptor) | Inhibition of adenylyl cyclase |
| $CB_2$ (cannabinoid receptor) | Inhibition of adenylyl cyclase |
| CXCR1 (chemokine receptor) | Activation of $G_i$ and $G_{16}$ |
| CXCR2 (chemokine receptor) | Activation of $G_i$ and $G_{16}$ |
| CXCR3 (chemokine receptor) | Activation of $G_i$ |
| CXCR4 (chemokine receptor) | Activation of $G_i$ |
| CCR1 (chemokine receptor) | Activation of $G_i$ |
| CCR2 (chemokine receptor) | Activation of $G_i$ |
| CCR3 (chemokine receptor) | Activation of $G_i$ |
| CCR4 (chemokine receptor) | Activation of $G_i$ |
| CCR5 (chemokine receptor) | Activation of $G_i$ |
| CCR6 (chemokine receptor) | Activation of $G_i$ |
| CCR7 (chemokine receptor) | Activation of $G_i$ |
| CCR8 (chemokine receptor) | Activation of $G_i$ |
| $CX_3CR1$ (chemokine receptor) | Activation of $G_i$ |
| $CCK_1$ (cholecystokinin receptor) | Activation of $G_{q/11}$ |
| $CCK_2$ (cholecystokinin receptor) | Activation of $G_{q/11}$ |
| $CRF_1$ (corticotropin-releasing factor receptor) | Stimulation of adenylyl cyclase |
| $CRF_2$ (corticotropin-releasing factor receptor) | Stimulation of adenylyl cyclase |
| $D_1$ (dopamine receptor) | Increased cAMP |
| $D_2$ (dopamine receptor) | Decreased cAMP |
| $D_3$ (dopamine receptor) | Decreased cAMP |
| $D_4$ (dopamine receptor) | Decreased cAMP |
| $D_5$ (dopamine receptor) | Increased cAMP |
| $H_2$ (histamin receptor) | Stimulation of adenylyl cyclase |
| $5-HT_1$ (5-hydroxytryptamine receptor) | Activation of $G_{i/o}$ |
| $5-HT_5$ (5-hydroxytryptamine receptor) | Activation of $G_s$ |
| $5-HT_6$ (5-hydroxytryptamine receptor) | Activation of $G_s$ |
| $5-HT_7$ (5-hydroxytryptamine receptor) | Activation of $G_s$ |
| $mt_1$ (melatonin receptor) | Inhibition of cAMP |
| $MT_2$ (melatonin receptor) | Inhibition of cAMP |
| $P2Y_1$ (P2Y receptor) | Activation of $G_{q/11}$ |
| $P2Y_2$ (P2Y receptor) | Activation of $G_{q/11/i/o}$ |

(continued)

| Receptor | Effect mediated by agonist |
|---|---|
| P2Y$_4$ (P2Y receptor) | Activation of G$_{q/11/i}$ |
| P2Y$_6$ (P2Y receptor) | Activation of G$_{q/11}$ |
| P2Y$_{11}$ (P2Y receptor) | Activation of G$_{q/s}$ |
| OP$_1$ (opinoid receptor) | Inhibition of adenylyl cyclase |
| OP$_2$ (opinoid receptor) | Inhibition of adenylyl cyclase |
| OP$_3$ (opinoid receptor) | Inhibition of adenylyl cyclase |
| DP (prostanoid receptor) | Stimulation of adenylyl cyclase |
| EP$_2$ (prostanoid receptor) | Stimulation of adenylyl cyclase |
| EP$_3$ (prostanoid receptor) | Inhibition of adenylyl cyclase |
| EP$_4$ (prostanoid receptor) | Stimulation of adenylyl cyclase |
| IP (prostanoid receptor) | Stimulation of adenylyl cyclase |
| sst$_1$ (somatostatin receptor) | Inhibition of adenylyl cyclase |
| SST$_2$ (somatostatin receptor) | Inhibition of adenylyl cyclase |
| sst$_3$ (somatostatin receptor) | Inhibition of adenylyl cyclase |
| sst$_4$ (somatostatin receptor) | Inhibition of adenylyl cyclase |
| sst$_5$ (somatostatin receptor) | Inhibition of adenylyl cyclase |
| PAC$_1$ (VIP and PACAP receptor) | Stimulation of adenylyl cyclase |
| VPAC$_1$ (VIP and PACAP receptor) | Stimulation of adenylyl cyclase |
| VPAC$_2$ (VIP and PACAP receptor) | Stimulation of adenylyl cyclase |

However, a GPCR of interest may naturally not couple to a signal pathway involving cyclic nucleotides. Those GPCR may also be used in the context of the present invention as their coupling may be altered, e.g. by using a G protein chimera. A G protein chimera is assembled from portions of different G proteins containing additional modifications and combines sequences of various α subunits within one protein. This fusion protein of different G proteins may be designed by fusing the G$_\alpha$ receptor recognition region of one G protein to the G$_\alpha$ effector activation region of another G protein. The aim is to provide a G protein chimera which receives signals from G protein coupled receptors which may be coupled to a G$_{\alpha q}$ or G$_{\alpha 12}$ protein but switches on the signal transduction pathway of G$_{\alpha s}$ or G$_{\alpha i}$ proteins to which the receptor is naturally not coupled to. This 'recoupling' of receptors has the advantage that any GPCR may be used in the test system, methods or uses of the invention, particularly in high-throughput screening. This technique is exemplified in WO 2004/055048 or WO 2001/036481.

A further aspect of the present invention relates to a method of detecting a change of an intracellular cyclic nucleotide level, the method comprising the steps of:

(a) subjecting the test system according to the present invention to an agent or a condition; and
(b) detecting the activity of the ion channel protein, wherein a change in activity of the ion channel protein is indicative of a change in intracellular cyclic nucleotide level.

The test system may be as defined in any of the embodiments of the test system described above.

The test system may be subjected to an agent under circumstances suitable to detect a measurable activity of the insect nucleotide-activated ion channel, including a suitable temperature, chemical environment (buffers, pH-value, etc.) as well as a suitable concentration of the agent and an appropriate time of contact. Alternatively, the test system may be subjected to a certain condition. This certain condition may be a condition that leads to a response of the test system by changing the intracellular cyclic nucleotide level. Such a condition may comprise a physical condition (temperature, pH) or a state (starvation, shear stress).

In general, steps a) and b) of the method of the invention are well known to the skilled person. However, in the following, they will be briefly summarized and/or further illustrated by examples. The skilled person will understand that the described method may be modified depending on the respective protein of the invention to be isolated, the cell used for cell culture, etc.

In brief, the method may be carried out as follows: A suitable cell expressing the insect nucleotide-activated ion channel protein and optionally additional components of the test system as defined above, is maintained (e.g. cultured) under suitable conditions and contacted (incubated) with the agent. After a time sufficient to allow for an effect on the cell, or simultaneously with the contacting, the activity of the insect nucleotide-activated ion channel protein is detected, wherein the detection of the activity is indicative of a change of an intracellular cyclic nucleotide level. The detection of the activity of the insect nucleotide-activated ion channel protein may be carried out with means of detection used in the test system. The method is further exemplified in Examples 1 and 2.

A still further aspect of the present invention relates to a method of identifying an agent capable of modifying an intracellular cyclic nucleotide level, the method comprising the steps of:

(a) contacting the test system according to the present invention with an agent; and
(b) detecting the activity of the ion channel protein, wherein a change in activity of the ion channel protein is indicative of the capability of the agent to modify an intracellular cyclic nucleotide level.

The test system may be as defined in any of the embodiments of the test system described above.

"Modifying an intracellular cyclic nucleotide level" in the context of the present invention means a change, either increase or decrease, of an intracellular cyclic nucleotide level in comparison to a control. This means that the intracellular cyclic nucleotide level of the cell contacted with the (test) agent is significantly lower or higher, respectively, than that of the control (e.g. the same cell not contacted with the (test) agent, or the same cell solely contacted with another agent known to induce a change in the intracellular cyclic nucleotide level). The person skilled in the art knows statistical procedures to assess whether two values are significantly different from each other, such as Student's t-test or chi-squared test (see also Examples for suitable statistical methods).

The activity of the insect nucleotide-activated ion channel protein (which is indicative of a change of an intracellular cyclic nucleotide level) which correlates e.g. with the current or ion flux through the channel, or the intracellular concentration of an ion relating to the channel may increase to at least 110 %, preferably to at least 125 %, more preferably to at least 150 %, 160 %, 170 %, 180 % or 190 %, still more preferably to at least 200 % and most preferably to least 300 % of the control determined as $\Delta$%. Alternatively, the activity of the insect nucleotide-activated ion channel protein (which is indicative of a change of an intracellular cyclic nucleotide level) may decrease to at least 80 %, preferably to at least 70 %, more preferably to at least 60 %, 50 %, 40 %, 30 %, 20% or 10 %, still more preferably to at least 5 % and most preferably to at least 0 % of the control determined as $\Delta$%. Methods for detecting the activity of the insect nucleotide-activated ion channel protein are well known to the skilled person and further exemplified above and in the examples.

The methods of the invention may be used for screening for a medicament for preventing and/or treating a disease involving GPCR dysfunction. If used for screening purposes the agent tested may be either a known GPCR ligand or agent, the function of which is still unknown or not yet related to a GPCR. Accordingly, the method may be used for the identification of a new GPCR ligand. Alternatively, known GPCR ligands may be tested for their efficiency or agents may be tested for their capability in modifying an intracellular cyclic nucleotide level, as defined above using the methods of the invention.

The ligand may be provided in the form of a chemical compound library. Chemical compound libraries include a plurality of chemical compounds and have been assembled from any of multiple sources, including chemically synthesized molecules and natural products, or have been generated by combinatorial chemistry techniques. They are especially suitable for high-throughput screening. They may be comprised of chemical compounds of a particular structure or compounds of a particular creature such as an animal. In the context with the present invention the chemical compound library is preferably a library comprising small molecules, proteins and peptides.

In a preferred embodiment the agent is a receptor ligand, preferably an agonist or an antagonist. In accordance with the present invention, the test system of the invention may be used for the identification of a ligand for GPCRs, particularly an agonist or an antagonist, e.g. by switching on or switching off the downstream signal pathway, i.e. the generation of a second messenger, such as a cyclic nucleotide. This cyclic nucleotide can subsequently activate the insect cyclic nucleotide-activated ion channel protein, whose activation can be detected by the means of detection, as defined above. Thus, a modification of the cyclic nucleotide may be detected in order to detect binding of a ligand to a GPCR. If it is searched for an agonist, the agonist may be identified by the activation of the downstream signal pathway. On the other hand, an antagonist might be identified by switching off the downstream signal transduction. In case of an antagonist, it might be necessary to use the combination of an agonist and a potential antagonist in order to detect the deactivation of agonist-induced signal transduction by the antagonist.

The agonist may be a full, partial or inverse agonist. Full agonists activate the insect nucleotide-activated channel to a maximal extent. Agents having a lower effect than a full agonist are called partial agonist, since they stimulate signal transduction but to a lesser extent than a full agonist. An inverse agonist is a ligand which produces an effect opposite to that of the agonist by occupying the same receptor. Especially in systems in which the GPCR is over-expressed inverse agonists can be identified. They reduce the basal level of the measurable or detectable signal upon binding to the receptor.

For the identification of full, partial and inverse agonists the method can be performed by carrying out steps (a) and (b). Hereto the test system is incubated with the agent in a suitable environment (buffer, temperature, etc.) and for a time sufficient to detect or measure the activity of the insect nucleotide-activated ion channel protein. If the test compound is any kind of agonist, the concentration should be high enough to be effective, i.e. to induce an activity, but should not exceed the toxicity level.

If an agonist was identified it can for example be further characterized by using the test system as a full or partial agonist. This can be done by concentration response curves. Hereunto, the test system is incubated with increasing concentrations of the test agent and the signal is plotted against the concentration of the agonist. The resulting curve can be analyzed using the following equation:

$$S([C]) = S_{min} + (S_{max} - S_{min}) \cdot [C]/([C] + EC_{50})$$

[C] = concentration of the test agent C
S([C]) = signal at a particular concentration of test agent C
$S_{min}$ = basal or minimal signal
$S_{max}$ = maximal signal
$EC_{50}$ = concentration of test agent C mediating a half maximal signal

The test agent is then characterized by the $EC_{50}$ and $S_{max}$. If $S_{max}$ is as high as $S_{max}$ induced with a full agonist, the test agent is also a full agonist. In contrast, if it is lower, the test agent is a partial agonist. An alternative method is exemplified in Example 1.

In addition, this method of screening can also be performed by carrying out steps (a) and (b) for the identification of antagonists. Since antagonists do usually not mediate the production of a measurable or detectable signal, they may not be identified directly. Therefore, the binding of an antagonist is identified by blocking the effect of a known agonist. The test system is incubated with the test agent and an agonist in a suitable environment (buffer, temperature, etc.) and for a time sufficient to detect or measure an activity of the insect nucleotide-activated ion channel protein. If the test agent is any kind of antagonist the concentration should be high enough to inhibit the activity of the insect nucleotide-activated ion channel protein mediated by the agonist. The concentration of the test agent and the agonist should not exceed the toxicity level.

If an antagonist was identified it can be further characterized by using the test system. Hereunto, the test system is incubated with increasing concentrations of the test agent in the presence of a defined concentration of an agonist. The signal is plotted against the concentration of the antagonist. The resulting curve can be analyzed using the in the following equation:

$$S([B]) = S_{min} + (S_{max} - S_{min}) \{1 - [B]/([B] + IC_{50})\}$$

[B] = concentration of the test agent B (blocker = antagonist)
S([B]) = signal at a particular concentration of test agent B
$S_{min}$ = basal or minimal signal
$S_{max}$ = maximal signal
$IC_{50}$ = concentration of test agent B mediating a half maximal inhibition

The blocker constant $K_B$ can be calculated according to:

$$K_B = IC_{50} \{1 + [A]/EC_{50}\}$$

[A] = concentration of the agonist

$EC_{50}$ = concentration of the agonist mediating a half maximal signal

The test agent is then characterized by the $K_B$. In general drugs with low $K_B$-values are preferred, since less substance is needed to block or inhibit the same signal.

In a preferred embodiment of the invention the methods of the invention may be used in a high-throughput assay. In such an assay the method of the present invention is advantageously automated and miniaturized; in particular by using miniaturized wells and microfluidics controlled by a roboter. A high-throughput assay or high-throughput screening (HTS), is a method for scientific experimentation especially used in drug discovery and relevant to the fields of biology and chemistry.

HTS allows a researcher to effectively conduct millions of biochemical, genetic or pharmacological tests in a short period of time, often through a combination of modem robotics, data processing and control software, liquid handling devices, and sensitive detectors. Through this process one can rapidly identify active agents which modulate a particular biomolecular pathway; particularly a substance modifying an intracellular cyclic nucleotide level.

In essence, HTS uses an approach to collect a large amount of experimental data on the effect of a multitude of substances on a particular target in a relatively short time. A screen, in this context, is the larger experiment, with a single goal (usually testing a scientific hypothesis), to which all this data may subsequently be applied.

For HTS cells comprising insect cyclic nucleotide-activated channel protein and probably one or more additional component(s) involved in the change of cyclic nucleotide level in a cell, or a nucleic acid coding for the same may be seed in a tissue plate, such as a multi well plate, e.g. a 96-well plate. Then the cell in the plate is contacted with the test substance for a time sufficient to stimulate and generate a suitabel detectable signal as defined above. The test substance may be different from well to well across the plate. After incubation time has passed to allow generation of the signal, measurements are taken across all the plate's wells, either manually or by a machine.

Manual measurements may be necessary when the researcher is using patch-clamp to (for example) detect changes in the current of the insect cyclic nucleotide-activated channel, looking for effects not yet implemented in automated routines. Otherwise, a specialized automated analysis machine can run a number of experiments on the wells (such as analysing light of a particular frequency or a high-throughput patch-clamp measurement). In this case, the machine outputs the result of each experiment e.g. as a grid of numeric values, with each number mapping to the value obtained from a single well.

Depending upon the results of this first assay, the researcher can perform follow up assays within the same screen by using substances similar to those identified as active (i.e. modifying an intracellular cyclic nucleotide level) into new assay plates, and then rerunning the experiment to collect further data, optimize the structure of the chemical agent to improve the effect of the agent on the cell.

Automation is an important element in HTS's usefulness. A specialized robot is often responsible for much of the process over the lifetime of a single assay plate, from creation through final analysis. An HTS robot can usually prepare and analyze many plates simultaneously, further speeding the data-collection process.

Examples for apparatuses suitable for HTS in accordance with the present invention comprise a Fluorometric Imaging Plate Reader (FLIPR™; Molecular Devices), FLEXstation™ (Molecular Devices), Voltage Ion Probe Reader (VIPR, Aurora Biosciences), Attofluor® Ratio Vision® (ATTO).

Yet, another aspect of the invention relates to a kit comprising

i) a cell or means for producing a cell, the cell as defined in the context of any of the above embodiments, and
ii) means for detecting activity of the ion channel protein as defined in the context of any of the above embodiments.

Therefore, a kit of the invention comprises a cell of the test system, as defined above, for example in a suitable medium or buffer. The medium or buffer, in which the cell may be incorporated, may be frozen or in a liquid state. Optionally or alternatively, the kit may comprise a nucleotide sequence encoding the insect cyclic nucleotide-activated ion channel protein, which may be used to produce the cell according to the invention, and/or one or more additional components, as defined above (e.g. a nucleic acid coding for the fluorescent marker or one or more GPCRs or G proteins), either in a liquid or lyophilized form. Additionally, the kit may include buffers and reagents for transfecting a cell with the provided nucleotide sequence(s). This may also include reagents for maintaining and selecting the cells after transfection, as e.g. an appropriate selection reagent such as an antibiotic. Furthermore, the kit comprises means for detecting activity of the ion channel. As defined above, these means may include an ion specific fluorescent marker (or a nucleic acid coding therefore), or a derivative thereof such as an ester. The kit may also include buffer and/or detergent to load the cell with the fluorescent marker. The fluorescent marker may be included as a salt or dissolved in a solvent such as e.g. DMSO, water or buffer. Optionally, the kit may include at least one microelectrode, patch-electrode or high-throughput patch-electrode, optionally including suitable electrode solution, as defined above. Finally, a manual with information and instruction for use and handling of the kit and its ingredients may be enclosed.

A further aspect of the present invention relates to the use of a test system, as defined herein, for detecting a change of an intracellular cyclic nucleotide level. Alternatively or additionally, the test system may be used for identifying an agent capable of modifying an intracellular cyclic nucleotide level. In a preferred embodiment the test system is used in a high throughput assay. The use may be characterized as detailed in connection with the above embodiments of the test system and methods of the invention.

Finally and also in accordance with the present invention, an insect cyclic nucleotide-activated ion channel protein, capable of coupling to an insect odorant-sensitive receptor protein, or a functionally active derivative thereof, may be used for detecting the activity of the ion channel protein. This may be carried out either in any kind of cell, including an insect cell, or a cell membrane, as long as the cell (i) does not endogenously express an insect odorant-sensitive receptor, as defined herein, or (ii) does not comprise an insect odorant-sensitive receptor, as defined herein.

In the following, the present invention is illustrated by figures and examples which are not intended to limit the scope of the present invention.

**FIGURES**

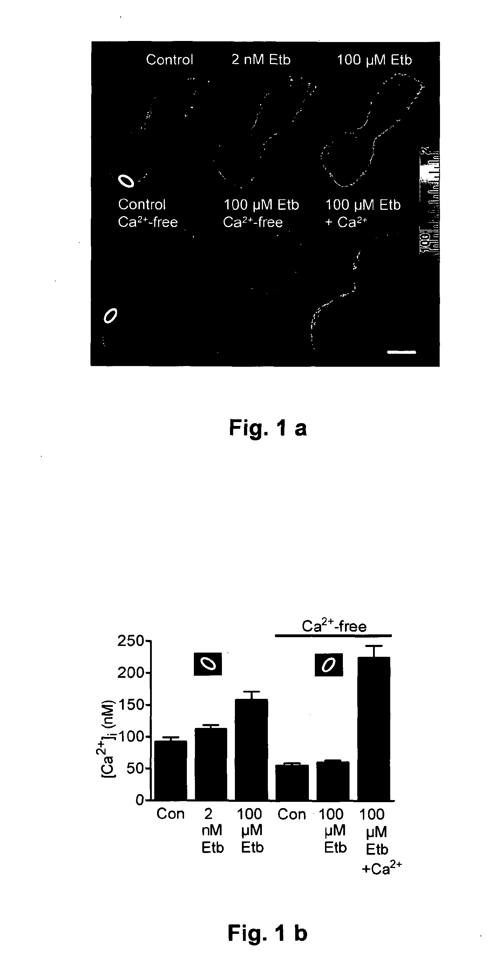

**Figure 1** shows that co-expressed Or22a and Or83b mediate ethyl butyrate-stimulated $Ca^{2+}$ influx.

**a**, Images of the free intracellular calcium concentration ($[Ca^{2+}]_i$ in nM) in HEK293 cells expressing Or22a and Or83b; top: before (Control) and one minute after application of ethyl butyrate (Etb) in a saline containing 1 mM $Ca^{2+}$; bottom: in a $Ca^{2+}$-free bath solution Etb-induced elevation of $[Ca^{2+}]$ is only detectable when $Ca^{2+}$ is coapplied with the odorant Etb (right). Scale bar, 10 $\mu$m. **b**, $[Ca^{2+}]_i$ measured in the regions indicated in **a** (*areas indicated by white ellipses).

**Figure 2** shows that co-expressed Or22a and Or83b mediate ethyl butyrate-stimulated ion currents.

**a, c,** Recordings (**a**) and maxima (**c**) of currents activated by Etb (100 nM; applied for 1 s, bar) under whole-cell voltage-clamp at -60 mV with 2 mM internal ATP and GTP (*top*) or ADP and GDP (*bottom*). Etb elicits a rapidly developing, transient inward current ($I_i$, ionotropic current) which overlaps with a slowly activating and slowly decaying current that requires the presence of ATP/GTP ($I_m$, metabotropic current). $n = 18$; *, $P < 0.05$; ***, $P < 0.001$; Student's t test (**c**). **b, d**, Recordings (**b**) and maxima (**d**) of currents activated as in **a** from outside-out patches. Without ATP/GTP there is only the initial response of $I_i$ (*bottom*), while in the presence of ATP/GTP both $I_i$ and $I_m$ are activated (*top*). $n = 15$; *, $P < 0.05$; Student's t test (**d**). **e**, Current responses to the indicated pulse protocol obtained in the absence (Control) and in the presence of 100 $\mu$M Etb for 1 min (control currents subtracted, Etb-Control). **f**, Responses to ramp voltage protocols ($\pm$100 mV in 400 ms) for Etb-induced currents (at indicated Etb concentrations, control currents subtracted, *top*) and for currents recorded before (Control) and after application of 10 $\mu$M benzaldehyde (Bea) or 100 nM Etb, respectively (*bottom*).

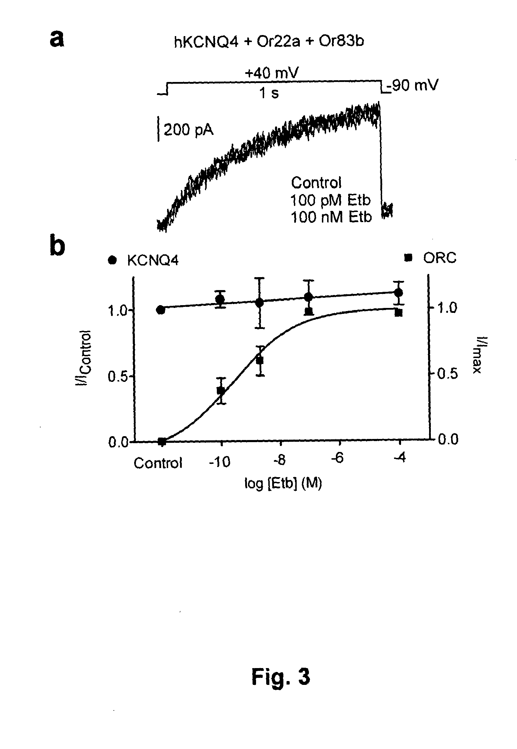

**Figure 3** shows testing for odour signal transduction via the G protein $G_q$.

**a**, Odour stimulation by Etb of Or22a and Or83b coexpressed in HEK293 cells with hKCNQ4, a voltage-gated potassium channel that reports Gq protein activation by an alteration of its activity, does not affect the KCNQ4 current activated by a voltage step to +40 mV. Linearly voltage-dependent currents were subtracted using a P/n protocol. **b**, While increasing Etb concentrations produce an increasing olfactory receptor current (ORC, black squares, data normalized to maximum response, the fitted curve is described by $EC_{50} = 256$ pM and Hill coefficient = 0.41) the KCNQ4 current at +40 mV (circles) remains unaffected (data presented as means and s.e.m.; n = 6).

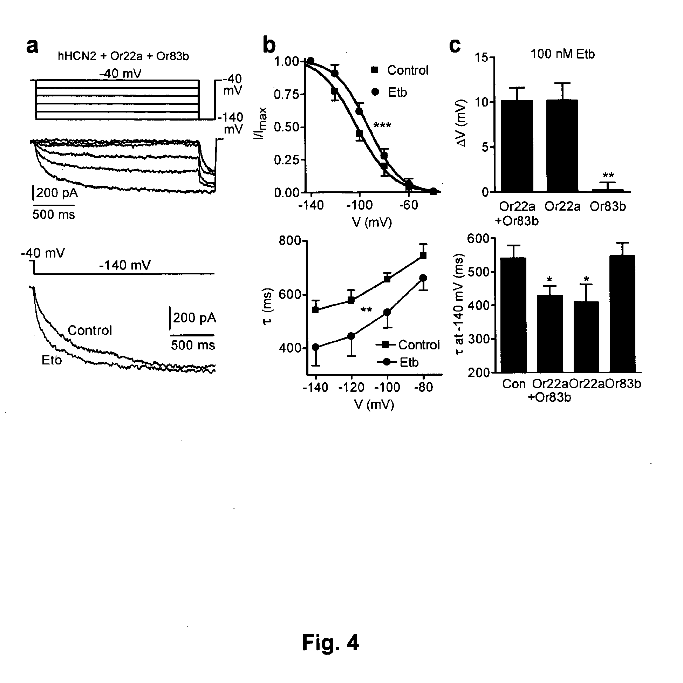

**Figure 4** shows testing for odour signal transduction via the G protein $G_s$.

**a**, Or22a, Or83b and the hHCN2 channel, which is activated by hyperpolarization and modulated by cAMP, were coexpressed in HEK293 cells. The current family (top) was activated under control conditions according to the given pulse protocol. Odour stimulation (2 min 100 nM Etb) accelerated the current time course (bottom). **b**, Normalized maximal currents (top) and activation time constants (bottom) as a function of test potential of hHCN2 channels coexpressed with Or22a before and after stimulation with 100 nM Etb. n = 7; **, $P < 0.01$; ***, $P < 0.0001$; ANOVA test. **c**, Effect of 100 nM Etb on the depolarizing shift of the hHCN2 current half-maximal activation potential ($\Delta$V, top) and activation time constant at -140 mV (bottom) in cells coexpressing the indicated ORs. n = 7; *, $P < 0.05$; **, $P < 0.01$; Student's t test.

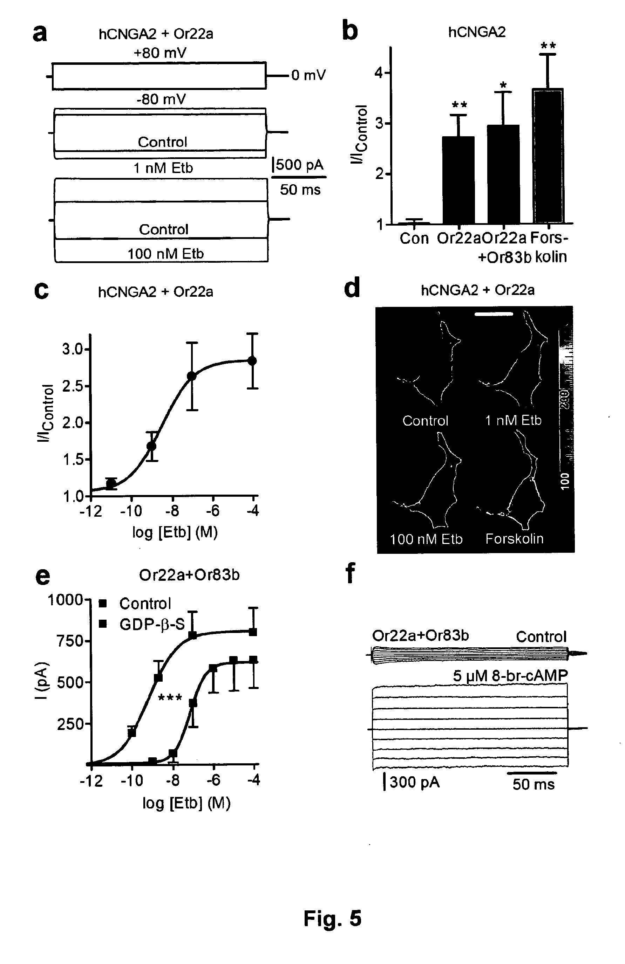

**Figure 5** shows testing for odour signal transduction via $G_s$ protein signalling.

**a**, Whole-cell current responses in a cell coexpressing the cyclic nucleotide-gated ion channel hCNGA2 with Or22a before and after application of 1 and 100 nM Etb. **b**, Relative increase in hCNGA2 current at -80 mV induced by 100 nM Etb when hCNGA2 was expressed alone (Con) or coexpressed with the indicated Ors. The right bar indicates the stimulating effect of 10 $\mu$M forskolin in cells expressing hCNGA2 alone. $n = 5$; *, $P < 0.05$; **, $P < 0.01$; Student's t test. **c**, Concentration-response curve for Etb-induced currents in cells expressing hCNGA2 and Or22a, measured at -80 mV and normalized to controls ($n = 5$). The continuous curve is a Hill fit ($EC_{50}$ = 3 nM, Hill coefficient = 0.54). **d**, Free $[Ca^{2+}]_i$ (in nM) in a HEK293 cell expressing hCNGA2 and Or22a before (Control) and 5 minutes after application of Etb or 10 $\mu$M forskolin, respectively. Scale bar, 10 $\mu$m. **e**, Concentration-response curves for Etb-induced currents under control conditions and with 500 $\mu$M GDP-$\beta$-S in the pipette to disrupt G protein activation ($n = 9$, Control; $n = 11$, GDP-$\beta$-S; ***, $P < 0.001$; ANOVA). The continuous curves are Hill fits described by $EC_{50}$ values of 677 pM and 70 nM and Hill coefficients of 0.61 and 1.1 for the control and GDP-$\beta$-S, respectively. **f**, Current families according to the pulse protocol of Fig. 2e before (Control) and after bath application of 8-bromo-cAMP (8-br-cAMP) in a cell expressing Or83b and Or22a.

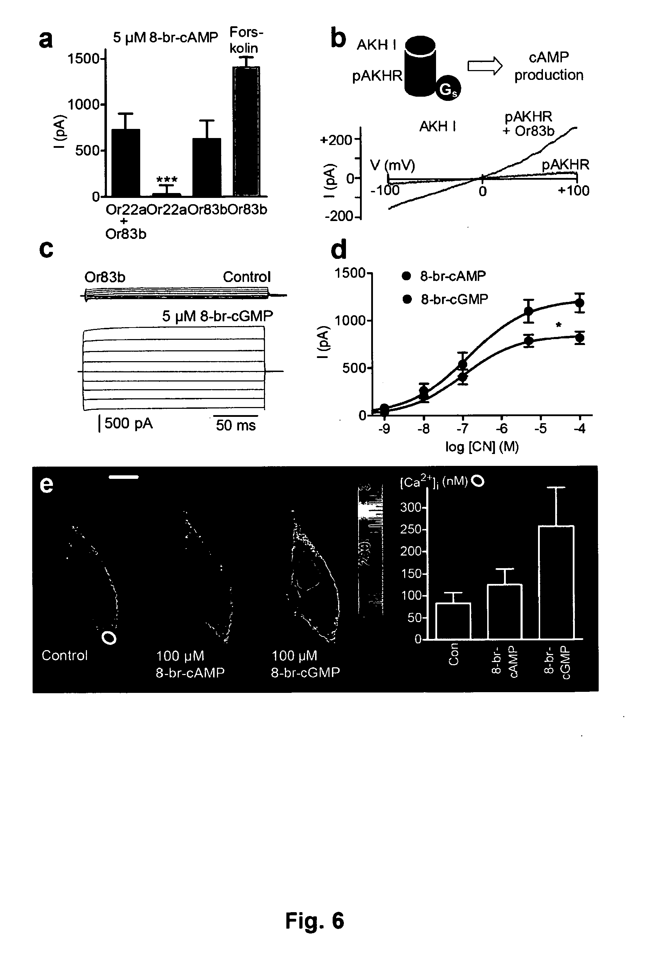

**Figure 6** shows that cyclic nucleotides activate ORs.

**a**, Maximal inward currents at -100 mV activated by 5 $\mu$M 8-bromo-cAMP or 50 $\mu$M forskolin in cells expressing ORs as indicated ($n = 7$; ***, $P < 0.001$; Student's t test). **b**, Scheme of endogeneous cAMP production by AKH 1-induced stimulation of the $G_s$ protein-activating *Periplaneta* adipokinetic hormone receptor pAKHR (*top*) and current responses to ramp voltage protocols ($\pm$100 mV in 400 ms) recorded from cells expressing pAKHR either alone or together with Or83b upon application of 10 nM AKH I (difference curves: 5 min AKH I - before AKH I, *bottom*). The bath solution contained the PLC inhibitor U73122 (5 $\mu$M) to depress $G_q$ protein related signalling. **c**, Current families recorded in a cell expressing Or83b before (Control) and after stimulation by 8-bromo-cGMP. **d**, Concentration-response curves for current activation by 8-bromo-cAMP and 8-bromo-cGMP in cells expressing Or83b (-100 mV; *, $P < 0.05$; ANOVA test). **e**, Free $[Ca^{2+}]_i$ (in nM) in a cell expressing Or83b under control conditions and 3 minutes after application of 8-bromo-cAMP and 8-bromo-cGMP (after 5 minutes of washing). The histogram shows $[Ca^{2+}]_i$ in a region at the lower pole of the cell (*area indicated by white ellipse*).

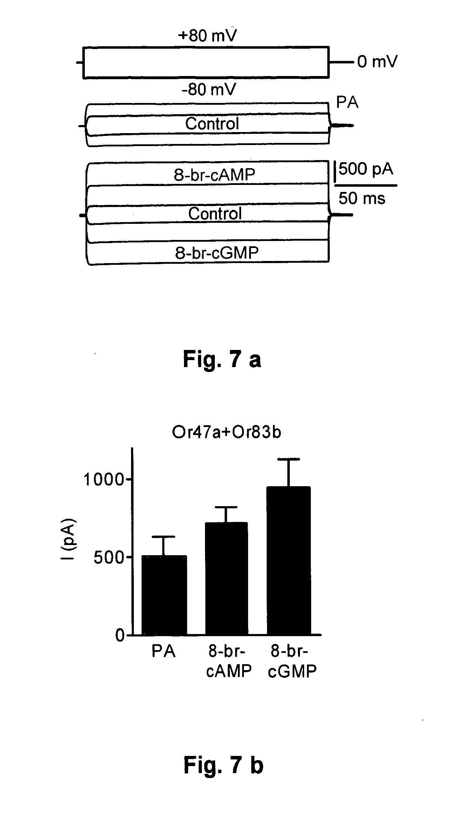

**Figure 7** shows that cyclic nucleotides activate currents through the receptor complex Or47a/Or83b.

**a**, Superimposed current responses in cells expressing Or47a and Or83b to the indicated pulse protocol (top) obtained in the absence (Control) and in the presence of 100 $\mu$M pentyl acetate (PA, middle) or 100 $\mu$M 8-bromo-cAMP and 8-bromo-cGMP (bottom), respectively. **b**, Maximal inward currents at -100 mV activated by 100 $\mu$M PA, 8-bromo-cAMP and 8-bromo-cGMP (n = 8).

**Figure 8** shows that modification of a putative Or83b pore region alters ion permeability. **a**, The wild type (WT) Or83b amino acids $V^{394}$ and $L^{398}$ were deleted in a mutant termed "GYG" (*left panel*). Family of currents activated by 8-bromo-cAMP in a cell expressing the Or83b GYG mutant (recording protocol as in Fig. 2 e; "0" indicates the baseline) (*right panel*). **b**, Degree of current rectification of 8-br-cAMP-activated (5 $\mu$M) wild-type and mutant channels, determined as absolute value of currents obtained at +100 mV relative to those at -100 mV. The bath/pipette solutions contained in mM: 140 $Na^+$/ 140 $K^+$ ($Na^+/K^+$) and 10 $Ca^{2+}$/ 140 $K^+$ ($Ca^{2+}/K^+$). $n = 7$; *, $P < 0.05$; Student's t test. **c**, Superimposed current responses to ramp voltage protocols ($\pm$100 mV in 400 ms) upon stimulation of HEK293 cells expressing WT or GYG with 5 $\mu$M 8-br-cAMP under conditions as in **b**. **d**, Scheme of odorant receptor and channel function. Stimulated by an odorant such as Etb, Or22a activates the channel protein Or83 by an ionotropic and a metabotropic pathway. The direct activation of Or83b by Or22a (light grey arrow; fast short) leads to a fast and transient ion conductance. The metabotropic pathway includes activation of $G_s$ proteins by Or22a. The $G_s\alpha$-subunit stimulates the adenylyl cyclase (AC) thereby increasing cAMP production. cAMP slowly activates a long-lasting nonselective cation conductance. Opening of the Or83b channel that harbours the TVVGYLG motif leads to $Na^+/Ca^{2+}$ influx and membrane depolarization.

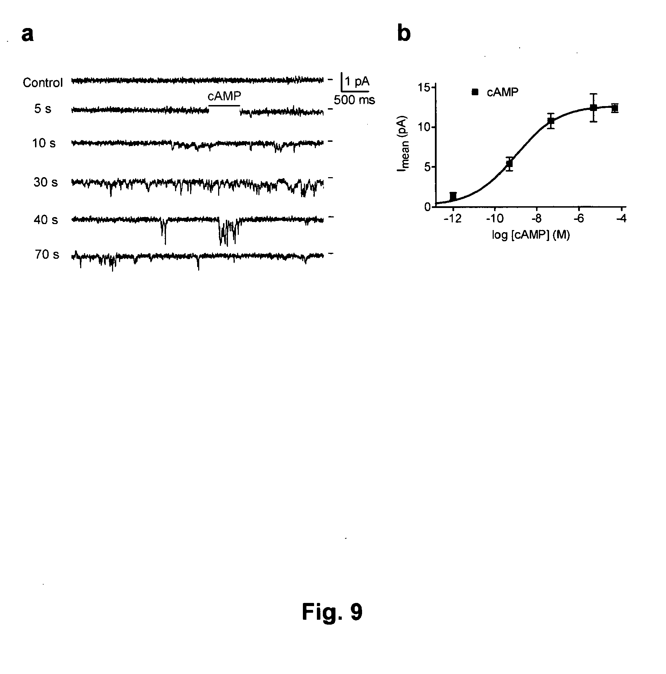

**Figure 9** shows cAMP sensitivity of Or83b in HEK293 cells.

**a**, Currents recorded at -60 mV in an inside-out patch of a cell expressing Or83b before (Control) and after application of 500 pM cAMP (grey bar) into the solution facing the cytosolic side of the membrane. Black bars, zero current level. **b**, Concentration-response curves for mean currents of inside-out patches from HEK293 cells expressing Or83b activated by cAMP. Mean currents were calculated from recordings as shown in a at the peak of the response to cAMP (duration 5 s, -60 mV; $n = 10$). The fitted curve is characterized by $EC_{50}$ = 1 nM and Hill coefficient = 0.42.

## EXAMPLES

## EXAMPLE 1: Investigation of the mechanism of OR activation in HEK cells

From worm to man, many odorant signals are perceived by the binding of volatile ligands to odorant receptors (OR) that belong to the G-protein-coupled receptor (GPCR) family. They couple to heterotrimeric G-proteins most of which induce cAMP production. This second messenger then activates cyclic nucleotide-gated ion channels to depolarise the olfactory receptor neuron, thus providing a signal for further neuronal processing. Recent findings, however, have challenged this concept of odorant signal transduction in insects, since their ORs, which lack any sequence similarity to other GPCRs (Benton et al., *PLoS Biol.* **4,** e20 (2006)), are composed of conventional ORs (e.g., Or22a), dimerised with a ubiquitously expressed chaperone protein, such as Or83b in *Drosophila* (Larsson et al., Neuron 43, 703-14 (2004)). Or83b has a structure akin to GPCRs, but has an inverted orientation in the plasma membrane (Benton et al., supra; Wistrand et al., Protein Sci. 15, 509-21 (2006). However, G-proteins are expressed in insect olfactory receptor neurons (Rützler et al., J. Comp. Neurol. 499, 533-45 (2006), and olfactory perception is modified by mutations affecting the cAMP transduction pathway (Martin et al., J. Comp. Physiol. [A] 187, 359-70 (2001)). Accordingly, inventors investigated the mechanism of OR activation in HEK cells.

MATERIAL AND METHODS:

*Cell culture and transfection:*

HEK293 cells were cultured as described by Wicher and colleagues (Wicher et al., J. Biol. Chem. 281, 3227-36 (2006)). In brief, HEK293 cells were cultured at a density of ~$2*10^4$ per 35-mm dish and transfected with 1 μg pcDNA3-DOR22a-GFP and 1 μg pcDNA3-DOR83b-GFP (Neuhaus, et al., Nat. Neurosci. 8, 15-17 (2005); DNAs were a generous gift of Dr. E. Neuhaus and Dr. H. Hatt) using Roti-Fect transfection kit (Roth, Karlsruhe, Germany). In some experiments we transfected cells with pcDNA3-DOR22a-GFP or pcDNA3-DOR83b-GFP alone. We also examined the properties of DOR47a, which was cloned, using standard molecular biology methods into a pcDNA3.1(-) expression vector. To test for putative G-protein coupling, cells were transfected with the respective OR-DNA together with 1.5 μg hHCN2/pcDNA3 (provided by M. Biel, Munich, Germany), with 1 μg hKCNQ4/pcDNA3.1 or with 1 μg hCNGA2/pCMV6-XL4 (Origene, Rockville, MD, USA). For elevation of cAMP level via AKH receptor stimulation cells were transfected with 1 μg pAKHR/pcDNA3.1.

For electrophysiological experiments we only used cells showing GFP fluorescence (when illuminated at 470 nm) as indicator of putative OR expression.

In addition, we also generated N-terminal GFP fusion proteins of Or22a and Or83b. These were constructed according to standard molecular biology protocols. Briefly, the OR genes were cloned into a pAcGFP1-C1 mammalian expression vector (Clontech laboratories Inc, Palo Alto, CA, USA) in frame with the GFP coding sequence, with no intervening stop codon, thus fusing the AcGFP molecule to the N-terminus of the OR genes. These constructs were then transfected as outlined above.

Site-directed PCR mutagenesis was used to introduce two mutations in the putative pore region of Or83b. Oligonucleotide primers were designed to replace the wild-type TVVGYLG motif with TVGYG. The mutation was subsequently confirmed via sequencing.

*Electrophysiology:*

Ion currents in HEK293 cells were measured with the patch-clamp technology as described (Wicher et al, J. Biol. Chem. 281, 3227-36 (2006)). In brief, ion currents in HEK293 cells were measured at room temperature using whole-cell patch-clamp with appropriate compensation of series resistance and of capacitive currents. Additional experiments were performed in the outside-out configuration. Current measurements and data acquisition were performed using an EPC9 patch-clamp amplifier controlled by PatchMaster software (both HEKA Elektronik, Lambrecht, Germany). Patch-clamp pipettes were fabricated from borosilicate capillaries. Pipettes for whole-cell recordings had resistances of 2-4 MΩ for excised-patch recordings the pipette resistance was up to 15 MΩ.

The standard pipette solution contained (in mM) 140 KCl, 4 NaCl, 2.2 CaCl$_2$, 2 Mg-ATP, 0.05 Na-GTP, 5 EGTA, 10 HEPES (pH 7.3), and the standard bath solution contained (in mM) 135 NaCl, 5 KCl, 1 CaCl$_2$, 1 MgCl$_2$, 10 HEPES, 10 glucose (pH 7.4). To differentiate between ionotropic and metabotropic pathways of receptor activation (Fig. 2a-d) the pipette solution contained either 2 mM ATP plus 2 mM GTP or 2 mM ADP plus 2 mM GDP (both for whole-cell and outside-out configuration). For the preparation of Cl$^-$-free solutions the chloride salts were substituted by gluconate salts. The pipette Cs$^+$ solution contained (in mM) 140 CsCl, 1 Mg-ATP, 10 EGTA, 10 HEPES (pH = 7.3), and the Cs$^+$ bath solution contained (in mM) 140 CsCl, 15 glucose, 15 HEPES (pH = 7.4). The Ca$^{2+}$-free bath solution contained (in mM) 140 NaCl, 15 glucose, 15 HEPES, and the Na$^+$-free bath solution contained (in mM) 140 NMDG-Cl, 10 CaCl$_2$, 10 glucose, 15 HEPES.

The permeability ratios were calculated according to

$$P_{Na}{:}P_K = [K^+]_i/[Na^+]_o * \exp(V_{rev}F/RT) \qquad (1)$$

and

$$P_{Ca}{:}P_K = [K^+]_i/4[Ca^{2+}]_o * \exp(V_{rev}2F/RT) \qquad (2)$$

where $P_{ion}$ are the permeabilities, $[ion]_i$ and $[ion]_o$ are the internal and external activity of the respective ion, and $V_{rev}$ is the reversal potential (Hille, B. Ion channels of excitable membranes (Sinauer Associates, Sunderland, 2001)). To rule out an effect on endogenous HEK293 channels (Bugaj et al., J. Biol. Chem. 280, 16790-7 (2005)), Etb, 8-bromo-cAMP and 8-bromo-cGMP were first tested on non-transfected cells ($n$ = 5-7).

*Calcium imaging:*

Free intracellular $Ca^{2+}$ concentration ($[Ca^{2+}]_i$) was determined by fura-2 fluorescence as described (Messutat et al., Cell Calcium 30, 199-211. (2001)). In brief, cells were loaded with fura-2 by incubation in bath solution containing 2 $\mu$M fura-2/acetomethylester for 20 min. Free intracellular $Ca^{2+}$ concentration ($[Ca^{2+}]_i$) was determined with the fluorescence ratio method. Excitation of fura-2 at 340 and 380 nm was performed with a monochromator (Polychrome II, T.I.L.L. Photonics, Gräfelfing, Germany) coupled via an epifluorescence condenser into an Axioskop FS microscope (Carl Zeiss, Jena, Germany) with a water immersion objective (LUMPFL 40xW/IR/0.8; Olympus, Hamburg, Germany). Emitted light was separated by a 400-nm dichroic mirror and filtered with a 420-nm long-pass filter. $[Ca^{2+}]_i$ was calculated according to the equation

$$[Ca^{2+}]_i = K_{eff}(R-R_{min})/(R_{max}-R). \qquad (3)$$

$K_{eff}$, $R_{min}$, and $R_{max}$ were determined using HEK293 cells permeabilized with 2 $\mu$M ionomycin and three solutions with different concentrations of free $Ca^{2+}$ ($Ca^{2+}$ free; 5 mM $Ca^{2+}$; 500 nM $Ca^{2+}$; the composition of the 500 nM solution was calculated with WEBMAXC v.2.20) (Patton et al., Cell Calcium 35, 427-31 (2004)). The values of $M_{eff}$, $R_{min}$, and $R_{max}$ were 3.72 $\mu$M, 0.38, and 5.9, respectively.

Fluorescence images were acquired using a cooled CCD camera controlled by TILLVision 4.0 software (T.I.L.L. Photonics). The resolution was 640x480 pixel in a frame of 175x130 $\mu$m (40x/IR/0.8 objective). Image pairs were obtained by excitation for 100 ms at 340 nm and 380 nm; background fluorescence was subtracted.

*Radioreceptor assay:*

Monolayers of HEK293 cells were incubated for 30 min in HEPES Ringer's buffer containing 1 mM isobutylmethylxanthine and stimulated with 100 nM Etb for 15 min. The reaction was stopped with 96% ethanol, and after evaporation, 50 mM Tris/4 mM EDTA (pH 7.5) was added. The cAMP content of the cellular extracts was measured by a cAMP [3H] assay system (GE Healthcare, Buckinghamshire, UK) using the affinity of a purified cAMP binding protein and a charcoal separation step according to the instructions of the manufacturer. Proteins were measured in parallel samples and intracellular cAMP concentration was expressed in pmol/mg cell protein.

*Substance application:*

With the exception of GDP-$\gamma$-S, which was applied via the patch pipette, all substances were applied to the bath using either a bath perfusion system (BPS4 from ALA, New York, USA) or a rapid solution changer (RSC160 from Biologic, Claix, France) which were controlled by the PatchMaster software (HEKA Elektronik).

*Data analysis, presentation and statistics:*

Results were given as means $\pm$ standard error of mean (s.e.m.), n = number of cells. The evaluation of statistical significance of differences was performed with Student's t-test for testing one variable and with two-way ANOVA for testing two variables.

For data analysis the software IgorPro (WaveMetrics, Lake Oswego, OR, USA) or Prism 4 (Graph Pad Software, San Diego, CA, USA) were used.

*Chemicals:*

8-bromo-cAMP, 8-bromo-cGMP, benzaldehyde (Bea), ethyl butyrate (Etb), forskolin, GDP-β-S, KT5720 and pentyl acetate (PA) were obtained from Sigma (Taufkirchen, Germany); myristoylated PKA inhibiting peptide 14-22 amide (PKI1422) and U73122 from Calbiochem (Darmstadt, Germany); fura-2 and wheat germ agglutine from Invitrogen (Darmstadt, Germany).

RESULTS:

To investigate the mechanism of OR activation we performed electrophysiological and fluorescence optical experiments on heterologously expressed ORs (Wetzel et al., Proc. Natl. Acad. Sci. U S A 98, 9377-80 (2001); Katada et al, Biochem. Biophys. Res. Commun. 305, 964-9 (2003)) of the fruit fly (*Drosophila melarrogaster*). Coexpression of Or22a and Or83b in human embryonic kidney (HEK293) cells (confirmed by confocal micrographs of HEK293 cells transfected with GFP control plasmid, GFP-Or83b N-terminal fusion protein, and GFP-Or22a N-terminal fusion protein as well as rhodamine fluorescence imaging the rhodamine-labelled plasma membrane; membrane insertion of the OR proteins was demonstrated by colocalising the GFP signal with red fluorescence of the rhodamine-labelled plasma membrane) and stimulation with the odorant ethyl butyrate (Etb) (Dobritsa et al, Neuron 37, 827-41 (2003)) led to an increased intracellular $Ca^{2+}$ concentration ($[Ca^{2+}]_i$) as previously reported by Neuhaus et al. (supra). The rise in the $[Ca^{2+}]_i$ level was dependent on the Etb concentration and relied on the presence of extracellular $Ca^{2+}$ suggesting a $Ca^{2+}$ influx rather than a $Ca^{2+}$ release from intracellular stores (Fig. 1).

Under voltage-clamp control at -60 mV in the whole-cell patch-clamp configuration an inward current developed upon short Etb application (Fig. 2 a) while no current was observed when HEK293 cells were transfected with either Or22a or Or83b alone ($n$ = 6). The odour-induced current is carried by cations, as it remained unchanged in Cl⁻-free solutions (n = 5, not shown), and is composed of an ionotropic ($I_i$) and a metabotropic ($I_m$) component. $I_i$ which does not rely on the presence of internal ATP and GTP (Fig. 1a, *bottom*) activates rapidly and shows a fast decay (peak at 1 s, termination at 10 s, see Tab. 1). $I_m$ that relies on internal ATP and GTP (Fig. 2a, *top*) develops and decays considerably slower than $I_i$ (latency 10 s, peak at 60 s, termination at 80 s, Tab. 1) but is considerably larger than $I_i$ (Fig. 2c, *top*).

Experiments with outside-out patches confirmed the whole-cell recording results (Fig. 2b, d, Tab. 2). The nature of the $I_i$ component is at present unclear but could result from a conformational change in the OR complex in response to odorant binding.

**Table 1.** Parameters characterizing currents activated by Etb application (100 nM, 1 s) in HEK293 cells expressing Or22a (C-terminal and N-terminal GFP fusion protein) and Or83b. Currents were measured in the whole-cell configuration at -60 mV in the presence of ATP and GTP (+) and in the presence of ADP and GDP (-). $I_i$, ionotropic current; $I_m$, metabotropic current; Decay time is the time at which the current response terminated. The latency of $I_i$ was < 0.5 s.

| Or 22a | ATP GTP | $I_i$ Peak size (pA) | $I_i$ Peak time (s) | $I_i$ Decay time (s) | $I_m$ Latency (s) | $I_m$ Peak size (PA) | $I_m$ Peak time (s) | $I_m$ Decay time (s) | n |
|---|---|---|---|---|---|---|---|---|---|
| C | + | 100 ± 23 | 1.10 ± 0.03 | 10.5 ± 0.7 | 10 ± 2 | 291 ± 54 | 58 ± 4 | 79 ± 5 | 16 |
| N | + | 98 ± 23 | 1.16 ± 0.06 | 10.2 ± 0.9 | 11 ± 3 | 311 ± 51 | 62 ± 9 | 80 ± 8 | 8 |
| C | - | 133 ± 51 | 1.13 ± 0.02 | 10.8 ± 0.6 | 18 ± 8 | 77 ± 7 | 43 ± 9 | 68 ± 8 | 6 |
| N | - | 132 ± 38 | 0.90 ± 0.17 | 10.4 ± 0.9 | 24 ± 5 | 51 ± 6 | 44 ± 6 | 63 ± 9 | 11 |

**Table 2.** Parameters characterizing currents activated by Etb application (100 nM, 1 s) in HEK293 cells expressing Or22a (C-terminal GFP fusion protein) and Or83b. Currents were measured in the outside-out configuration at -60 mV in the presence of ATP and GTP (+) and in the presence of ADP and GDP (-). $I_i$, ionotropic current; $I_m$, metabotropic current; Decay time is the time at which the current response terminated. The latency of $I_i$ was < 0.5 s.

| Or 22a | ATP GTP | $I_i$ Peak size (pA) | $I_i$ Peak time (s) | $I_i$ Recov. (s) | $I_m$ Latency (s) | $I_m$ Peak size (pA) | $I_m$ Peak time (s) | $I_m$ Recov. (s) | n |
|--------|---------|------|------|------|------|------|------|------|---|
| C | + | 1.3 ± 0.3 | 1.13 ± 0.11 | 5.2 ± 1.1 | 10 ± 2 | 1.7 ± 0.4 | 33 ± 4 | 45 ± 7 | 10 |
| C | - | 1.6 ± 0.3 | 1.38 ± 0.23 | 4.5 ± 1.2 | 11 ± 3 | 0.5 ± 0.2 | 36 ± 8 | 58 ± 9 | 6 |

Further experiments were focused on the metabotropic current response. The Etb-induced currents showed no marked voltage-dependence except for a weak outward rectification (Fig. 2 e,f). The instantaneous current responses following steps in the membrane potential suggest that the odorant-induced channels are constitutively active. No current was detected using symmetrical $Cs^+$ solutions (140 mM in pipette and bath solution) (not shown, $n$ = 6). The current size depended on odorant concentration, saturating at about 100 nM Etb (Fig. 2f, *top*), while the half-maximal effect was obtained at around 700 pM (Fig. 2 e). Part of the receptors seem to be active even in the absence of Etb since the Or22a-inhibitor benzaldehyde (Bea) (Pelz et al., J. Neurobiol. 66, 1544-63 (2006)) reduced the current recorded under control conditions (Fig. 2f, *bottom; n* = 6).

To test which G-proteins are involved in metabotropic odorant signalling we coexpressed Or22a with ion channels reporting G-protein activation (Wicher et al., J. Neurophysiol. 95, 2314-25 (2006)). ORs either couple to $G_s$ proteins thereby leading to elevated cAMP levels or to Gq proteins leading to $IP_3$ formation (Barry, J. Gen. Physiol. 122, 247-50 (2003)). For testing putative Gq protein coupling we coexpressed human KCNQ4 channels that are inhibited upon activation of endogenous Gq proteins. Application of Etb at various concentrations had no effect on the hKCNQ4 current although Etb produced olfactory receptor currents (Fig. 3). This indicates that OR activation by Etb does not lead to Gq protein activation. For testing putative $G_s$ protein coupling we first coexpressed human HCN2 channels that report changes in cyclic nucleotide concentrations (Ludwig et al., Embo J. 18, 2323-9 (1999)). Increasing intracellular cAMP concentration due to $G_s$ protein activation causes a depolarising shift of the steady-state activation curve and an acceleration of activation kinetics (Ludwig, supra). These modifications of HCN2 current characteristics were observed after Etb stimulation when these channels were coexpressed with Or22a plus Or83b, with Or22a alone, but not with Or83b alone (Fig. 4). Second, we coexpressed the cAMP-sensitive CNGA2 type of human cyclic nucleotide-gated (CNG) channels (Dhallan et al., Nature 347, 184-7 (1990)). In cells expressing Or22a plus Or83b or Or22a alone Etb caused a concentration-dependent rise in CNGA2 current (Fig. 5 a-c). Cells solely expressing CNGA2 did not respond to Etb, but to the adenylyl cyclase activator forskolin (Fig. 5 b). Since CNG channels are also permeable to $Ca^{2+}$ (Craven & Zagotta, Annu. Rev. Physiol. 68, 375-401 (2006)), we visualized the stimulation of CNGA2 channels by $Ca^{2+}$ imaging. Etb application produced an increase in $[Ca^{2+}]_i$ that was most prominent near the plasma membrane (Fig. 5 d). These data suggest that odorant stimulation of the "true" olfactory receptor Or22a increases the intracellular cAMP concentration via a $G_s$ pathway. Finally, a radioreceptor assay revealed a significantly elevated cAMP level of cells expressing Or22a and Or83b upon Etb stimulation (from 1.28 ± 0.06 (Control) to 1.65 ± 0.10 pmol/mg protein (100 nM Etb); $n$ = 3 experiments; $P$ < 0.05; Student's t test). The Etb-concentration dependence of cAMP production reported by CNGA2 currents (Fig. 5 c) was similar to that of receptor current stimulation (Fig. 2f, 5e).

Since the G protein-mediated signals are considerably slower than those arising from direct receptor activation (Fig. 2 a) we asked whether the involvement of G proteins enhances the odour sensitivity of the signalling system. We thus tested whether the non-hydrolysable G protein inhibitor GDP-β-S, intracellularly applied via the patch pipette, may reduce the Etb sensitivity. Compared with the Etb response under control conditions ($EC_{50}$ = 677 pM, Hill coefficient = 0.61) the presence of GDP-β-S caused a considerable shift of the Etb concentration dependence ($EC_{50}$ = 70 nM, Hill coefficient = 1.1, Fig. 5 e). This indicates that the ionotropic pathway of receptor activation requires significantly higher odour concentrations than the metabotropic pathway.

The cAMP signal originating from Or22a stimulation may subsequently initiate the activation of an ion channel complex requiring Or83b. If this were true, artificial elevation of the cAMP level in HEK293 cells expressing Or22a and Or83b should mimic the effect of Etb. Indeed, bath application of the membrane-permeable cAMP analogue 8-bromo-cAMP induced a membrane current similar to that produced by Etb (Fig. 5 f). Moreover, 8-bromo-cAMP induced a similar current in cells expressing Or83b alone (Fig. 6 a). 8-bromo-cAMP failed, however, to elicit a current response in cells solely expressing Or22a (Fig. 6 a). Stimulation of cAMP production by forskolin yielded large current responses of twice the size as obtained with 8-bromo-cAMP supporting the notion that cAMP serves as activating ligand for Or83b (Fig. 6 a). To illustrate that the current activated by 8-bromo-cAMP is identical to the current elicited by Etb, cells expressing Or22a and Or83b were first stimulated with 8-bromo-cAMP at a high concentration of 100 $\mu$M and subsequently treated with 100 $\mu$M Etb. Etb failed to further increase the current under these conditions (+1.6 $\pm$ 3.8% of current induced by 8-bromo-cAMP). To further test the notion that elevated intracellular cAMP levels lead to activation of Or83b, we next coexpressed Or83b with the *Periplaneta* adipokinetic hormone receptor (pAKHR) which is known to activate $G_s$ proteins (Wicher et al., 2006, supra). Stimulation of pAKHR with the adipokinetic hormone I (AKH I) induced a current (294 $\pm$ 64 pA at -100 mV, n = 10) but failed to do so when only AKHR was expressed (12 $\pm$ 24 pA, n = 6; Fig. 6 b).

The results reported so far for the metabotropic odour response indicate that the *Drosophila* olfactory receptor proteins Or22a and Or83b form a complex serving as both GPCR and ion channel. To clarify the role of cAMP in channel activation we tested whether an activity of a cAMP-dependent protein kinase (PKA) was necessary. Ion currents elicited by 8-bromo-cAMP stimulation of Or83b-expressing cells were not significantly altered by preincubation with the PKA inhibitors KT5720 (10 $\mu$M) or PKI1422 (1 $\mu$M; *n* = 6; not shown). Thus, channel phosphorylation by PKA can be excluded as activation mechanism.

Ion channels activated or modulated by cAMP such as CNG or HCN channels are also sensitive to cGMP (Craven & Zagotta, supra). In Or83b-expressing HEK293 cells 8-bromo-cGMP elicited a current similar to that obtained upon 8-bromo-cAMP application (Fig. 6 c). The concentration-response relations for both cyclic nucleotides show that 8-bromo-cGMP is a significantly better agonist than 8-bromo-cAMP for current activation (Fig. 6 d) and for the elevation of $[Ca^{2+}]_i$ (Fig. 6 e).

Ion selectivity of the channels formed by Or83b was assayed by changing the composition of bath solutions. By measuring the reversal potentials ($V_{rev}$) in $Ca^{2+}$-free and $Na^+$-free bath solutions, respectively, permeability ratios were determined according to eqs (1) and (2): $P_{Na}:P_K = 1.1 \pm 0.1$ from $V_{rev}$ of +4.0 $\pm$ 1.1 mV ($Ca^{2+}$-free, *n* = 7) and $P_{Ca}:P_K = 5.4 \pm 0.2$ from $V_{rev}$ of+1.1 $\pm$ 0.4 mV ($Na^+$-free, *n* = 7). Or83b thus forms a nonselective cation channel characterized by the permeability sequence $P_{Ca}:P_{Na}:P_K = 5.4:1.1:1$.

To test whether cyclic nucleotides also activate olfactory receptors containing OR proteins different from Or22a, Or83b was coexpressed with Or47a. Cells responding to the Or47a agonist pentylacetate (PA) (Hallem et al., Cell 117, 965-79 (2004)) were also responsive to 8-bromo-cAMP and 8-bromo-cGMP indicating that OR complex activation by cyclic nucleotides is not a phenomenon restricted to Or22a (Fig. 7).

Comparing the results for activation and ion selectivity of Or83b channels with those of CNG and HCN channels (both nonselective cation channels that pass monovalent cations) reveals similarities but also differences. The activation of Or83b is similar to that of CNG channels. Furthermore, $Ca^{2+}$ ions permeate Or83b and CNG channels (Kaupp & Seifert, Physiol. Rev. 82, 769-824 (2002)) much more readily than HCN channels (Craven & Zagotta, supra). However, while $Cs^+$ easily permeates CNG channels, it blocks Or83b moderately and HCN potently (Craven & Zagotta, supra).

In green algae two GPCRs form light-gated ion channels, the proton conducting channelrhodopsin-1 (Nagel, G. et al., Science 296, 2395-8 (2002)) and the nonselective cation conducting channelrhodopsin 2 (Nagel, G. et al., Proc. Natl. Acad. Sci. U S A 100, 13940-5 (2003)). Or83b only shows a low degree of sequence identity with these proteins (13.0 % amino acid identity with channelrhodopsin-2). Sequence comparison of Or83b with *Drosophila* ion channels, including CNG and HCN, also reveals a low degree of similarity. However, the motif TVVGYLG (393-399) (SEQ ID NO:1) located near the cytoplasmic border of the sixth predicted transmembrane helix of Or83b, is reminiscent of the selectivity filter motif TVGYG (SEQ ID NO: 2) in the pores of $K^+$ channels (Doyle et al., Science 280, 69-77. (1998)). Considering this motif as part of a putative selectivity filter, its modification might affect the ion permeability. Deletion of two residues in this motif as indicated in Fig. 8 a ("GYG mutation") altered the slight outward rectification of wild-type Or83b currents (Fig. 2f, 5f, 6b) into a moderate inward rectification (Fig. 8 a). Using both $Ca^{2+}$-free and $Na^+$-free bath solutions the ratio of currents measured at +100 mV and -100 mV was significantly reduced in GYG-expressing cells indicating an attenuated $K^+$ conductance (Fig. 4b). Additionally, the GYG mutation caused a shift in the reversal potentials. $V_{rev}$ was +24.8 $\pm$ 2.4 mV for $Ca^{2+}$-free (*n* = 5) and +16.5 $\pm$ 5.1 mV for $Na^+$-free (*n* = 7) bath solutions (Fig. 8 c). The permeability ratios determined according to eqs (1) and (2) were $P_{Na}:P_K = 2.5 \pm 0.3$ and $Pca:PK = 19.6 \pm 8.9$ indicating a reduction in $K^+$ permeability with respect to the wild type. These experiments demonstrate that Or83b forms ion channels in HEK293 cells and that the TVVGYLG motif is involved in controlling the channel's ion permeability (Fig. 8 d).

The pair of *Drosophila* olfactory receptor proteins Or22a/Or83b represents a typical insect OR. Homologues of Or83b have been identified in various other insect species. The design principle of insect ORs of being composed of an odorant-sensitive protein and of a protein finally transducing the chemical message into an electrical signal ensures very rapid recognition of high odour concentrations via the ionotropic pathway as well as a somewhat slower but prolonged and highly sensitive odour detection via the G protein-mediated signal amplification (Fig. 8 d).

CONCLUSION:

Here we show that application of odorants to mammalian cells coexpressing Or22a and Or83b results in nonselective cation currents activated via an ionotropic and a metabotropic pathway, and a subsequent increase in the intracellular $Ca^{2+}$ concentration. Expression of Or83b alone leads to functional ion channels not directly responding to odorants, but directly activated by intracellular cAMP or cGMP. Insect ORs thus form ligand-gated channels as well as complexes of odorant sensing units and cyclic nucleotide-activated nonselective cation channels. Thereby they provide rapid and transient as well as sensitive and prolonged odorant signalling.

**EXAMPLE 2: Evidence for the cAMP-sensitivity of *Drosophila* Or83b**

In the previous Example 1 we have shown by means of patch clamp investigations that stimulation of Or83b expressed in HEK293 cells with membrane-permeable cAMP and cGMP-analogues lead to an activation of ion currents through the cell membrane (Fig. 5f, Fig. 6 a, c and d). To get direct evidence for Or83b-activation by cAMP we performed excised-patch experiments in the inside-out configuration.

MATERIAL AND METHODS:

For this, HEK293 cells were cultured at a density of ~2*10^4 per 35-mm dish and transfected with 1 μg pcDNA3-DOR83b-GFP (Neuhaus et al., Nat Neurosci 8: 15-17, 2005.) using Roti-Fect transfection kit (Roth, Karlsruhe, Germany). For electrophysiological experiments we only used cells showing GFP fluorescence (when illuminated at 470 nm) as indicator of putative OR expression.

For electrophysiological studies, ion currents in HEK293 cells were measured at room temperature in the inside-out configuration. Current measurements and data acquisition were performed using an EPC9 patch-clamp amplifier controlled by PatchMaster software (both HEKA Elektronik, Lambrecht, Germany). Patch-clamp pipettes were fabricated from borosilicate capillaries. Pipettes for whole-cell recordings had resistances of up to 15 MΩ. The bath solution contained (in mM) 140 KCl, 4 NaCl, 2.2 $CaCl_2$, 2 Mg-ATP, 0.05 Na-GTP, 5 EGTA, 10 HEPES (pH 7.3), and the pipette solution contained (in mM) 135 NaCl, 5 KCl, 1 $CaCl_2$, 1 $MgCl_2$, 10 HEPES, 10 glucose (pH 7.4).

RESULTS:

Fig. 9a shows an example of recordings from an inside-out patch before and after application of 500 pM cAMP (bar); Fig. 9b shows the mean current induced in inside-out patches by application of cAMP at the indicated concentrations. The concentration-dependence of current production is described by an $EC_{50}$ of 1.1 nM. For comparison, the rather cAMP-sensitive vertebrate CNGA2 channels have an $EC_{50}$ of 39 μM (cGMP: 1 μM) (Dhallan et al., Nature 347: 184-181, 1990).

CONCLUSION:

Or83b is a highly sensitive sensor of cyclic nucleotides, particularly cAMP, as shown in the above excised-patch experiments in the inside-out configuration.

## Claims

1. A test system for detecting a cyclic nucleotide, the test system comprising:

   (a) a non-insect cell comprising an insect cyclic nucleotide-activated ion channel protein, capable of coupling to an insect odorant-sensitive receptor protein, or a functionally active derivative thereof; and
   (b) means for detecting the activity of the ion channel protein.

2. The test system of claim 1, wherein the cell does not comprise an insect odorant-sensitive receptor protein.

3. The test system of claim 1 or 2, wherein the non-insect cell is a vertebrate cell or cell line, preferably a mammalian cell or cell line, more preferably a mouse, rat, primate or human cell or cell line.

4. The test system according to any of claims 1 to 3, wherein the insect cyclic nucleotide-activated ion channel protein is insect Or83b or a homologue, or a functionally active derivative thereof, particularly wherein the insect cyclic nucleotide-activated ion channel protein is *Drosophila melanogaster* Or83b or a functionally active derivative thereof.

5. The test system according to any of claims 1 to 4, wherein the cyclic nucleotide is cAMP or cGMP.

6. The test system according to any of claims 1 to 5, wherein the means of detecting the activity of the ion channel protein comprise a marker, preferably a fluorescent marker, or means for detecting a current, an ion flux or ion, preferably a microelectrode, a patch-electrode, or a high-throughput patch-electrode.

7. The test system according to any of claims 1 to 6, wherein the non-insect cell further comprises one or more additional component(s) involved in the change of a cyclic nucleotide level in a cell, preferably wherein the one or more additional component is selected from a group consisting of:

(i) a G protein coupled receptor (GPCR), preferably wherein the GPCR is capable of coupling to a $G_s$ protein or to a $G_i$ protein, more preferably wherein the GPCR is capable of coupling to a $G_s$ protein;
(ii) a G protein or a G protein-chimera capable of coupling to adenylyl or guanyly cyclase, preferably wherein the G protein is a $G_s$ protein or a $G_i$ protein, more preferably wherein the G protein is a $G_s$ protein;
(iii) an adenylyl cyclase; and
(iv) a guanylyl cyclase.

8. A method of detecting a change of an intracellular cyclic nucleotide level, the method comprising the steps of:

(a) subjecting the test system according to any of claims 1 to 7 to an agent or a condition; and
(b) detecting the activity of the ion channel protein, wherein a change in activity of the ion channel protein is indicative of a change in intracellular cyclic nucleotide level.

9. A method of identifying an agent capable of modifying an intracellular cyclic nucleotide level, the method comprising the steps of:

(a) contacting the test system according to any of claims 1 to 7 with an agent; and
(b) detecting the activity of the ion channel protein, wherein a change in activity of the ion channel protein is indicative of the capability of the agent to modify an intracellular cyclic nucleotide level.

10. The method of claim 8 or 9, wherein the agent is a receptor ligand, particularly an agonist or an antagonist.

11. The method according to any of claims 8 to 10, wherein the method is used in a high throughput assay.

12. A kit comprising

(i) a cell or means for producing a cell, the cell as defined in any of claims 1 to 5 or 7, and
(ii) means for detecting activity of the ion channel protein as defined in claims 1 or 6.

13. Use of a test system according to any of claims 1 to 7 for detecting a change of an intracellular cyclic nucleotide level.

14. Use of a test system according to any of claims 1 to 7 for identifying an agent capable of modifying an intracellular cyclic nucleotide level.

15. Use of a test system according to claim 13 or 14 in a high throughput assay.

**Fig. 1 a**

**Fig. 1 b**

**Fig. 2**

**a** hKCNQ4 + Or22a + Or83b

**b**

Fig. 3

...

**Fig. 4**

**Fig. 5**

Fig. 6

**Fig. 7 a**

**Fig. 7 b**

Fig. 8

**Fig. 9**

Europäisches
Patentamt

European
Patent Office

Office européen
des brevets

**EUROPEAN SEARCH REPORT**

Application Number

EP 08 00 7140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2004 062273 A1 (GENOPTERA LLC [US]) 13 July 2006 (2006-07-13) | 1-3,5-15 | INV.<br>G01N33/50<br>G01N33/566 |
| Y | * paragraphs [0625], [0036], [0039], [0043], [0049], [-52131], [0140], [0144] - [0148]; claims 1-4 * | 4 | |
| Y | ----- <br>WO 03/016477 A (SENTIGEN CORP [US]; LEE KEVIN J [US]; NGUYEN THUY-AI T [US]; KLOSS BRI) 27 February 2003 (2003-02-27)<br>* abstract; claims 1,80 * | 4 | |
| Y | ----- <br>US 2005/053933 A1 (LEE KEVIN J [US] ET AL) 10 March 2005 (2005-03-10)<br>* abstract *<br>----- | 4 | |

TECHNICAL FIELDS
SEARCHED          (IPC)

G01N

The present search report has been drawn up for all claims

| Place of search | Date of completion of the search | Examiner |
|---|---|---|
| Munich | 11 February 2009 | Klee, Barbara |

EPO FORM 1503 03.82 (P04C01)

## ANNEX TO THE EUROPEAN SEARCH REPORT
## ON EUROPEAN PATENT APPLICATION NO.

EP 08 00 7140

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2009

| Patent document cited in search report | Publication date | Patent family member(s) | | Publication date |
|---|---|---|---|---|
| DE 102004062273 A1 | 13-07-2006 | NONE | | |
| WO 03016477 A | 27-02-2003 | CA | 2457226 A1 | 27-02-2003 |
| | | EP | 1578913 A2 | 28-09-2005 |
| | | JP | 2005510209 T | 21-04-2005 |
| | | MX | PA04001460 A | 17-02-2005 |
| US 2005053933 A1 | 10-03-2005 | US | 2008176322 A1 | 24-07-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005043158 A
- US 20050221426 A
- US 20030228633 A
- US 07166463 B
- US 07341836 B
- WO 03016477 A
- WO 2004100971 A
- US 5501958 A
- US 5334507 A
- US 5719036 A
- WO 2004055048 A
- WO 2001036481 A

**Non-patent literature cited in the description**

- **Dhallan et al.** *Nature,* 1990, vol. 347, 184-187
- **Benton et al.** *PLoS Biol.,* 2006, vol. 4, e20
- **Neuhaus et al.** *Nal. Neurosci.,* 2005, vol. 8, 15-17
- **Larsson et al.** *Neuron,* 2004, vol. 43, 703-14
- **Wistrand et al.** *Protein Sci.,* 2006, vol. 15, 509-21
- **Lundin et al.** *FEBS Lett.,* 2007, vol. 581, 5601-5604
- **Lundin et al.** *FEBS Letters,* 2007, vol. 581, 5601-5604
- **Rützler et al.** *J. Comp. Neurol.,* 2006, vol. 499, 533-45
- **Martin et al.** *J. Comp. Physiol. [A],* 2001, vol. 187, 359-70
- **Wicher et al.** *J. Biol. Chem.,* 2006, vol. 281, 3227-36
- **Neuhaus et al.** *Nat. Neurosci.,* 2005, vol. 8, 15-17
- **Bugaj et al.** *J. Biol. Chem.,* 2005, vol. 280, 16790-7
- **Messutat et al.** *Cell Calcium,* 2001, vol. 30, 199-211
- **Patton et al.** *Cell Calcium,* 2004, vol. 35, 427-31
- **Wetzel et al.** *Proc. Natl. Acad. Sci. U S A,* 2001, vol. 98, 9377-80
- **Katada et al.** *Biochem. Biophys. Res. Commun.,* 2003, vol. 305, 964-9
- **Dobritsa et al.** *Neuron,* 2003, vol. 37, 827-41
- **Pelz et al.** *J. Neurobiol.,* 2006, vol. 66, 1544-63
- **Wicher et al.** *J. Neurophysiol.,* 2006, vol. 95, 2314-25
- **Barry.** *J. Gen. Physiol.,* 2003, vol. 122, 247-50
- **Ludwig et al.** *Embo J,* 1999, vol. 18, 2323-9
- **Dhallan et al.** *Nature,* 1990, vol. 347, 184-7
- **Craven ; Zagotta.** *Annu. Rev. Physiol.,* 2006, vol. 68, 375-401
- **Hallem et al.** *Cell,* 2004, vol. 117, 965-79
- **Kaupp ; Seifert.** *Physiol. Rev.,* 2002, vol. 82, 769-824
- **Nagel, G. et al.** *Science,* 2002, vol. 296, 2395-8
- **Nagel, G. et al.** *Proc. Natl. Acad. Sci. U S A,* 2003, vol. 100, 13940-5
- **Doyle et al.** *Science,* 1998, vol. 280, 69-77
- **Neuhaus et al.** *Nat Neurosci,* 2005, vol. 8, 15-17
- **Dhallan et al.** *Nature,* 1990, vol. 347, 184-181

SEQUENCE LISTING

<110>  Max Planck Institute for Chemical Ecology

<120>  A test system for detecting intracellular cyclic nucleotide level
         and methods and uses thereof

<130>  M65536EP

<160>  2

<170>  PatentIn version 3.4

<210>  1
<211>  7
<212>  PRT
<213>  Artificial

<220>
<223>  Amino acids 393-399 of Drosophila melanogaster Or83b

<400>  1

Thr Val Val Gly Tyr Leu Gly
1               5


<210>  2
<211>  5
<212>  PRT
<213>  Artificial

<220>
<223>  Substitution for amino acids 393-399 of Drosophila melanogaster
         Or83b

<400>  2

Thr Val Gly Tyr Gly
1               5